# EUROPEAN PATENT APPLICATION

(11) **EP 2 366 702 A1**
(43) Date of publication of application: **21.09.2011**
(21) Application number: 10382060.1
(22) Date of filing: 18.03.2010
(51) Int. Cl.: C07D 413/04, A61K 31/4245, C07D 413/14, A61P 25/00, A61P 29/00, A61P 31/00, A61P 35/00

(54) **New oxadiazole derivatives**

(71) Applicant: Almirall, S.A., 08022 Barcelona (ES)
(72) Inventor: Aguilar Izquierdo, Nuria, 08980 Sant Feliu de Llobregat (ES); Fonquerna Pou, Silvia, 08980 Sant Feliu de Llobregat (ES); Taboada Martinez, Lorena, 08980 Sant Feliu de Llobregat (ES); Vidal Juan, Bernat, 08980 Sant Feliu de Llobregat (ES); Carrascal Riera, Marta, 08980 Sant Feliu de Llobregat (ES); Navarro Romero, Eloisa, 08980 Sant Feliu de Llobregat (ES)
(74) Representative: Elzaburu Marquez, Alberto

(57) **Abstract**

The present invention relates to a compound of formula (I), or a pharmaceutically acceptable salt or N-oxide thereof: 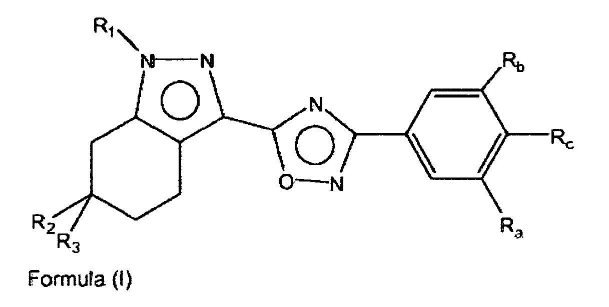 and its use in the treatment of diseases which are susceptible to improvement by sphingosine-1-phosphate (S1P1) receptor agonists.

## Description

The present invention relates to oxadiazole derivatives, to processes for their preparation and to pharmaceutical compositions containing them. These compounds are potent agonists of S1 P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

Sphingosine -1 phosphate (S1P) is a pleiotropic lipid mediator that exhibits a broad spectrum of biological activities, including cell proliferation, survival, lymphocyte trafficking, cytoskeletal organization, and morphogenesis. S1P is generated from endogenous sphingosine through phosphorylation by specific kinases, named sphingosine kinases 1 and 2. The levels of S1P in biological fluids and tissues are tightly regulated by the balance between its synthesis by sphingosine kinases and its degradation by S1 P lyase. This tight control is important since an excessive production of S1P has been associated to various pathological conditions, such as angiogenesis and vascular permeability changes in cancer, inflammation, myocardial infarction or transplant rejection.

Gene deletion studies and reverse pharmacology have provided evidence that most of the effects of S1P are mediated via five G-protein coupled receptor subtypes, named S1P1 to S1P5 (Brinkmann, Pharmacology & therapeutics 115:84-105, 2007). The interest on this family of receptors increased following the discovery that they were the pharmacological target of FTY720. This compound, a synthetic analog of a natural product derived from the fungus *Isaria sinclairii,* exhibited a peculiar immunomodulatory potential in vivo. When administered in vivo, it caused lymphopenia, due to the sequestration of lymphocytes from the blood into the lymph nodes and Peyer's patches. The close structural similarity of FTY720 to sphingosine, together with the discovery of the formation of phosphorylated FTY720 in vivo (FTY720P) prompted to speculate that FTY720-P could be acting as a mimetic of S1P. This proven to be the case and it was later on demonstrated that FTY-P binds 4 of the five known S1P receptors, namely S1P1, S1P3, S1P4 and S1P5.

Expression analysis identified S1 P1 as the dominant S1P receptor expressed on lymphocytes. Moreover, the transfer of S1P1-deficient T cells to normal mice led to the cells being sequestered in lymph nodes, as occurred with animals treated with fingolimod. These two facts strongly pointed out at S1 P1 as the main receptor involved in the lymphopenic effect of FTY-P in vivo (Baumruker et al, Exp. Opin. Invest. Drugs 2007; 16(3): 283-289). FTY720 is currently in phase III trials for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1 P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943) cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295), cardiovascular diseases (WO2004010987).

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes; systemic lupus erythematosis and Sjögrn's syndrome.

Rejection transplanted organs such as kidney, liver, heart, lung, pancreas, cornea and skin; graft-versus-hist disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies

Angiogenesis-related disorders that may be prevented or treated include but are not limited to hemangiomas, ocular neovascularization, macular degeneration or diabetic retinopathy.

Pain including neuropathic pain, that may be prevented or treated include but are not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation,trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction or complications from cardiac surgery or for improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain oxadiazoles are novel and potent agonists of S1 P1 and can therefore be used in the treatment or prevention of these diseases.

Thus the present invention is directed to new oxadiazole derivatives of formula (I) or pharmaceutically acceptable salts or N-oxides or solvates or deuterated derivative thereof: 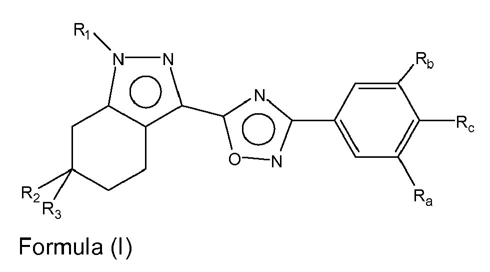
R¹ represents a hydrogen atom, a C₁₋₂ alkyl group or a C₃₋₄ cycloalkyl-C₁₋₂ alkyl group,
both R² and R³ represent a C₁₋₂ alkyl group;
R^{a} represents a hydrogen atom or a C₁₋₂ alkyl group;
R^{b} represents a hydrogen atom, a C₁₋₂ alkyl group, a C₃₋₄ cycloalkyl group, a carboxy group, a carbamoyl group or a -CF₃ group;
R^{c} represents a linear or branched C₂₋₅ alkyl group substituted with one substituent selected from a hydroxy group, a triazolyl group and a -P(O)(OH)₂ group; a -(CH₂)₍₁₋₂₎-C(O)OR' group; a -NR'R" group; a -(CH₂)(₁₋₂)-NR'R" group; a -(CH₂)(₁₋₂)-NHC(O)R" group; a -(CH₂)(₂₋₄)-NH-(CH₂)(₁₋₃)-NR'R" group; or a -O-(CH₂)(₂₋₃)-NR'R" group, wherein:
   R' represents a hydrogen atom or a C₁₋₂ alkyl group,
   R" represents a hydrogen atom; a C₅₋₆ cycloalkyl group substituted with a carboxy group; a 5- to 6-membered saturated heterocyclic ring containing one nitrogen atom and substituted with a carboxy(C₁₋₂alkyl) group; or a C₁₋₂ alkyl group optionally substituted by one or more substituents selected from halogen atoms, hydroxy groups, methyl groups, amino groups, carbamoyl groups and carboxy groups; or
   R' and R" together with the nitrogen atom to which they are attached form (a) a 4 to 6 membered saturated or unsaturated heterocyclic group, which contains, as heteroatom, one N atom and, optionally, one further N atom and which heterocyclic group is substituted with one or more substituents selected from a halogen atom, a hydroxy group, a methyl group, an oxy group and a carboxy group, or (b) a 5 membered heteroaryl group which contains, as heteroatom, one N atom and, optionally, one or more further N atoms and which heteroaryl group is optionally substituted with one substituent selected from an amino group and a carboxy group.

Further objectives of the present invention are to provide a method for preparing said compounds; pharmaceutical compositions comprising an effective amount of said compounds; compounds for use in the treatment of a human or animal body, in particular, for the treatment of pathological conditions or diseases susceptible to improvement by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases. The present invention further provides the use of the compounds in the manufacture of a medicament for the treatment of such pathological condition or diseases and methods of treatment of such pathological conditions or diseases susceptible to amelioration by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases comprising the administration of the compounds of the invention to a subject in need of treatment.

As used herein the term alkyl embraces linear or branched hydrocarbon radicals having 1 to 5 carbon atoms. Examples include methyl, ethyl, *n*-propyl, *i*-propyl, n-butyl, *i*-butyl, *t-butyl and* n-pentyl radicals.

As used herein, the term cycloalkyl embraces saturated carbocyclic radicals and, unless otherwise specified, a cycloalkyl radical typically has from 3 to 6 carbon atoms

Examples include cyclopropyl and cyclobutyl, cyclopentyl and cyclohexyl radical.

The 5 to 6-membered saturated heterocyclic ring which R" may represent is a saturated C₅-C₆ carbocyclic ring system in which one of the carbon atoms is replaced by a nitrogen atom. Thus, the 5 to 6-membered saturated heterocyclic ring is typically piperidyl or piperazinyl.

The 4 to 6-membered heterocyclic ring which R' and R" may form together with the nitrogen to which they are attached is a saturated or unsaturated C₄-C₆ carbocyclic ring system in which one of the carbon atoms is replaced by a nitrogen atom and optionally in which one further carbon atom is replaced by a nitrogen. Thus, said 4 to 6-membered saturated or unsaturated heterocyclic group contains, as heteroatoms, the N atom to which R' and R" are attached and 0 or 1 further N atoms.

Examples of 5 to 6-membered heterocyclic radicals include piperidyl, pyrrolidyl and piperazinyl. Piperidyl is preferred.

The 5 membered heteroaryl group which R' and R" may form together with the nitrogen to which they are attached comprises as heteroatoms, the N atom to which R' and R" are attached and 0 or 1 or more further N atoms, preferably 0, 1 or 2 further N atoms.

Examples of 5 membered heteroaryl radicals include pirrolyl, pirazolyl and triazolyl. Triazolyl is preferred.

In a carboxy(C₁₋₂ alkyl) group, the C₁₋₂ alkyl portion is preferably bonded to the 5 to 6-membered heterocyclic ring which R" may represent in the molecule depicted in formula (I).

When R¹ represents a C₃₋₄ cycloalkyl-C₁₋₂ alkyl group, it is to be understood that the C₁₋₂ alkyl portion is preferably bonded to the nitrogen atom of the molecule as depicted in formula (I).

As used herein, some of the atoms, radicals, moieties, chains or cycles present in the general structures of the invention are "optionally substituted". This means that these atoms, radicals, moieties, chains or cycles can be either unsubstituted or substituted in any posiition by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) -is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Typically, R¹ represents a methyl or ethyl group, preferably a methyl group.

Typically, both R² and R³ represent a methyl group.

Typically, R^{a} represents a hydrogen atom or a methyl group, preferably a methyl group

Typically, R^{b} represents a C₁₋₂ alkyl group, a C₃₋₄ cycloalkyl group or a -CF₃ group, preferably a methyl group or a cyclopropyl group, more preferably a methyl group.

Typically, R^{c} represents a linear or branched C₂₋₅ alkyl group substituted with a triazolyl group, a -(CH₂)₂-NR'R" group, a -(CH₂)₂NHC(O)R" group, or a -(CH₂)₂-NH-(CH₂)₂-NR'R" group, wherein
R' represents a hydrogen atom,
R" represents a hydrogen atom; a C₅₋₆ cycloalkyl substituted with a carboxy group; or a C₁₋₂ alkyl group optionally substituted by one or more substituents selected from halogen atoms, hydroxy groups, amino groups, carbamoyl groups and carboxy groups, or
R' and R" together with the nitrogen atom to which they are attached frorm (a) a 6 membered saturated or unsaturated heterocyclic group, which contains, as heteroatom, one N atom and, optionally, one further nitrogen atom and which heterocyclic group is substituted with one or two substituents selected from halogen atoms, hydroxy groups, methyl groups and carboxy groups, or (b) an unsubstituted triazolyl group.

When the 4 to 6 membered saturated or unsaturated heterocyclic group that R' and R" may form, together with the nitrogen atom to which they are attached, is substituted with an oxy group, there is preferably only one oxy group substituent.

Preferably, when R^{c} represents a -(CH₂)₂-NR'R' or a -(CH₂)₂-NH-(CH₂)₂-NR'R", R' and R" together with the nitrogen atom to which they are attached from an unsubstituted triazolyl group.

More preferably, R^{c} represents a -(CH₂)₂-NR'R" and R' represents a hydrogen atom and R" represents a C₁₋₂ alkyl group substituted by one or more substituents selected from a fluorine atom, a hydroxy group, a carbamoyl group and a carboxy group

In a preferred embodiment, both R¹, R², R³, R^{a} and R^{b} represent a methyl group and R^{c} represents a -(CH₂)₂-NR'R", wherein R' represents a hydrogen atom and R" represents a C₁₋₂ alkyl group substituted by one or more substituents selected from a fluorine atom, a hydroxy group, a carbamoyl group and a carboxy group.

In another preferred embodiment, R¹ represents a methyl group or an ethyl group; both R² and R³ represent methyl groups; R^{a} represents a hydrogen atom or a methyl group; R^{b} represents a hydrogen atom, a methyl group, a cyclopropyl group, a carboxy group or a carbamoyl group; R^{c} represents a linear or branched C₂₋₅ alkyl group substituted with one substituent selected from a hydroxy group, a triazolyl group and a -P(O)(OH)₂ group; a -(CH₂)₂-C(O)OR' group; a -NR'R" group; a -(CH₂)₂-NR'R" group, a -(CH₂)₂-NHC(O)R" group; a -(CH₂)₂-NH-(CH₂)₂-NR'R" group or a -O-(CH₂)₂-NR'R" group, wherein: R' represents a hydrogen atom, R" represents a hydrogen atom, a cyclopentyl group substituted with a carboxy group, a 6-membered saturated heterocyclic ring containing one nitrogen atom and substituted with a -CH₂COOH group, a C₁₋₂ alkyl group optionally substituted by one or more substituents selected from fluorine atoms, hydroxy groups, methyl groups, amino groups, carbamoyl groups and carboxy groups, or R' and R" together with the nitrogen atom to which they are attached from (a) a piperidine or piperazine ring which is substituted with one or more substituents selected from a fluorine atom, a methyl group, a hydroxy group, an oxy group and a carboxy group, or (b) a pyrazolyl or triazolyl group which is optionally substituted with one substituent selected from an amino group and a carboxy group.

Particular individual compounds of the invention include:
3-{2-Cyclopropyl-4-[5-(1-ethyl-6,6-dimethyl-4,5,6,7-tetrahydro-1H-indazol-3-yl)-1,2,4-oxadiazol-3-yl]phenyl}propanoic acid,
N-1-(2-{2,6-Dimethyl-4-[5-(1,6,6-trimethyl-4,5,6,7-tetrahydro-1H-indazol-3-yl)-1,2,4-oxadiazoi-3-yl]phenyl}ethyl)glycinamide,
N-1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-2-methylalaninamide,
N-2-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)glycinamide,
3-(3-{4-[2-(4,4-Difluoropiperidin-1-yl)ethyl]-3,5-dimethylphenyl}-1,2,4-oxadiazol-5-yl)-1,6,6-trimethyl-4,5,6,7-tetrahydro-1H-indazole,
(2-{2,6-Dimethyl-4-[5-(1,6,6-trimethyl-4,5,6,7-tetrahydro-1H-indazol-3-yl)-1,2,4-oxadiazol-3-yl]phenyl}ethyl)[2-(1H-1,2,4-triazol-1-yl)ethyl]amine,
4-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)piperazin-2-one,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-4-methylpiperidine-4-carboxylic acid,
N-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-L-serine,
3-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]-2,2-difluoropropanoic acid,
1-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]cyclopentanecarboxylic acid,
{4-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]piperidin-1-yl}acetic acid,
2-(2-Aminoethoxy)-5-[5-(1,6,6-trimethyl-4,5,6,7-tetrahydro-1H-indazol-3-yl)-1,2,4-oxadiazol-3-yl]benzoic acid,
2-(2-Aminoethoxy)-5-[5-(1,6,6-trimethyl-4,5,6,7-tetrahydro-1H-indazol-3-yl)-1,2,4-oxadiazol-3-yl]benzamide,
1-{4-[5-(1,6,6-Trimethyl-4,5,6,7-tetrahydro-1H-indazol-3-yl)-1,2,4-oxadiazol-3-yl]phenyl}piperidin-4-ol,
3-(3-{3,5-Dimethyl-4-[2-(1H-1,2,4-triazol-1-yl)ethyl]phenyl}-1,2,4-oxadiazol-5-yl)-1,6,6-trimethyl-4,5,6,7-tetrahydro-1H-indazole,
4-{2,6-Dimethyl-4-[5-(1,6,6-trimethyl-4,5,6,7-tetrahydro-1H-indazol-3-yl)-1,2,4-oxadiazol-3-yl]phenyl}-2-methylbutan-2-ol,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazol-3-amine,
3-(3-{3,5-Dimethyl-4-[2-(1H-1,2,4-triazol-3-yl)ethyl]phenyl}-1,2,4-oxadiazol-5-yl)-1,6,6-trimethyl-4,5,6,7-tetrahydro-1H-indazole,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-4-fluoropiperidine-4-carboxylic acid,
(2-{2,6-Dimethyl-4-[5-(1,6,6-trimethyl-4,5,6,7-tetrahydro-1H-indazol-3-yl)-1,2,4-oxadiazol-3-yl]phenyl}ethyl)phosphonic acid,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-4-carboxylic acid,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-3-carboxylic acid,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-5-carboxylic acid,
or a pharmaceutically acceptable salt or N-oxide thereof

Of outstanding interest are:
N-1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)glycinamide,
N-2-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)glycinamide,
(2-{2,6-Dimethyl-4-[5-(1,6,6-trimethyl-4,5,6,7-tetrahydro-1H-indazol-3-yl)-1,2,4-oxadiazol-3-yl]phenyl}ethyl)[2-(1H-1,2,4-triazol-1-yl)ethyl]amine,
3-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]-2,2-difluoropropanoic acid,
2-(2-Aminoethoxy)-5-[5-(1,6,6-trimethyl-4,5,6,7-tetrahydro-1H-indazol-3-yl)-1,2,4-oxadiazol-3-yl]benzoic acid,
3-(3-{3,5-Dimethyl-4-[2-(1H-1,2,4-triazol-1-yl)ethyl]phenyl}-1,2,4-oxadiazol-5-yl)-1,6,6-trimethyl-4,5,6,7-tetrahydro-1H-indazole,
(2-(2,6-Dimethyl-4-[5-(1,6,6-trimethyl-4,5,6,7-tetrahydro-1H-indazol-3-yl)-1,2,4-oxadiazol-3-yl]phenyl}ethyl)phosphonic acid,
1-(2-(2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-4-carboxylic acid, and
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-5-carboxylic acid.

Compounds of general formula (I) may be obtained following the synthetic scheme depicted in figure 1.

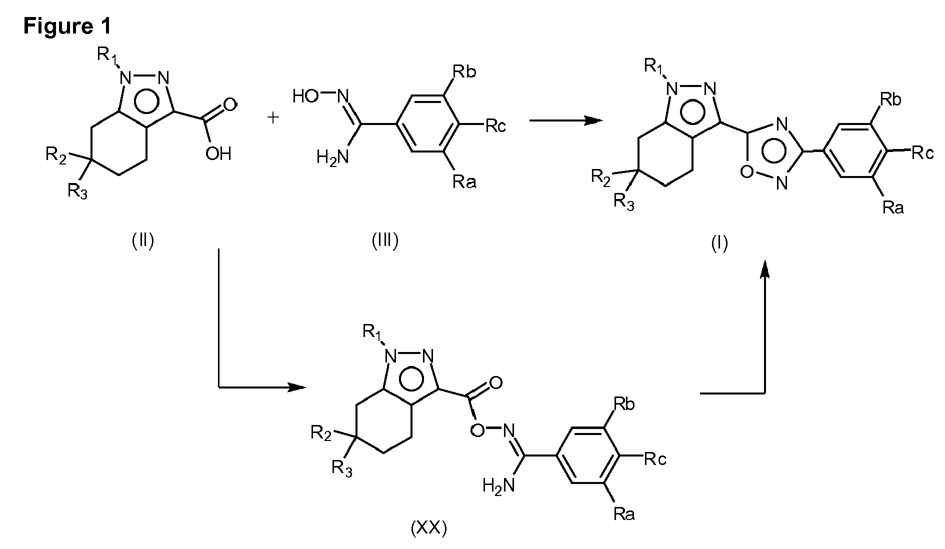

Compounds of general formula (I) may be prepared by the condensation of the tetrahidroindazole-3-carboxylic acid derivative of formula (II) with the corresponding carboximidamide derivative of formula (III) in a solvent such as dimethylformamide (DMF), N-methylpyrrolidone (NMP), tetrahydrofurane (THF), in the presence of one or more coupling agents such as 2-(1H-benzotriazole-1-yl)-1,2,3,3-tetramethyluronium tetrafluoroborate, N-(3-dimethyl aminopropyl)-N'-ethyl-carbodiimide, dicyclohexyl carbodiimide, 1-hydroxybenzotriazole, O-(benzotriazol-1-yl)-N,N,N',N'-tetramethyluroniium hexafluoro phosphate, carbonyl diimidazole, and optionally in the presence of a base such as triethylamine, Hünig's base, sodium hydride, potassium carbonate, at a temperature between 40 and 150°C and in a standard reactor.

An alternative method for the preparation of compounds of formula (I) may be done following a two steps synthesis. The first step is carried out by coupling intermediates of formula (II) and (III) with one or more coupling agent as described before to give intermediates of formula (XX) and then, in a second step, a subsequent cyclization in a solvent such as xylene, toluene, benzene, pyridine, DMF, dichloromethane, acetic acid, trifluoroacetic acid,. at room temperature or elevated temperatures, optionally in the presence of auxiliaries such as acid (e.g. trifluoroacetic acid, acetic acid, hydrochloric acid, etc.), bases (e.g., sodium hydride, sodium acetate, sodium carbonate, potassium carbonate, triethylamine, etc.), tetralkylammonium salts or water removing agents such as oxalyl chloride, a carboxylic acid anhydride, phosphoryl trichloride (POCl₃), tetrabutylammonium fluoride (TBAF), molecular sieves, etc.

Compounds of general formula (II) may be prepared following the synthetic scheme depicted in figure 2.

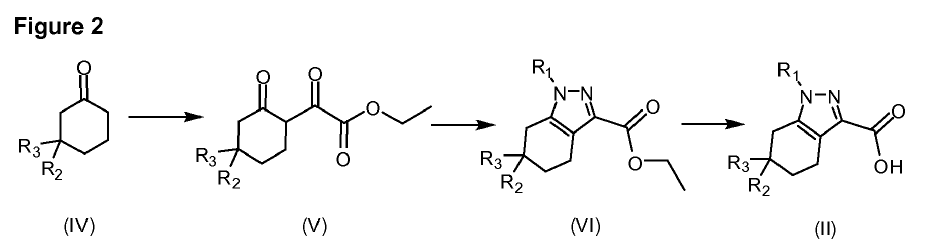

Intermediates of general formula (V) may be prepared by the reaction of the corresponding ketone derivatives of formula (IV) with diethyloxalate in a basic media such as sodium ethoxide or sodium hydride in a protic solvent such as ethanol and at a temperature between 20°C and the boiling point of the solvent. Ketones of general formula (IV) are either commercially available or may be prepared using synthetic methods known in the art.

Intermediates of general formula (VI) may be prepared by the condensation of the intermediates of formula (V) with the corresponding hydrazine in basic media such as triethylamine and in a protic solvent such as ethanol at a temperature between 20°C and the boiling point of the solvent.

Hydrolysis of the ethyl ester derivatives of formula (VI) in basic conditions with a base such as aqueous sodium hydroxide or litium hydroxide in a solvent such as methanol, ethanol or THF or a mixture of them at a temperature between 20 and 80°C or in acidic condicions with an acid such as HCl and a solvent such as water or ethanol or a mixture of them at a temperature between 20 and 80°C gives the acid intermediates of formula (II).

Intermediates of general formula (III) may be prepared following the synthetic scheme depicted in figure 3.

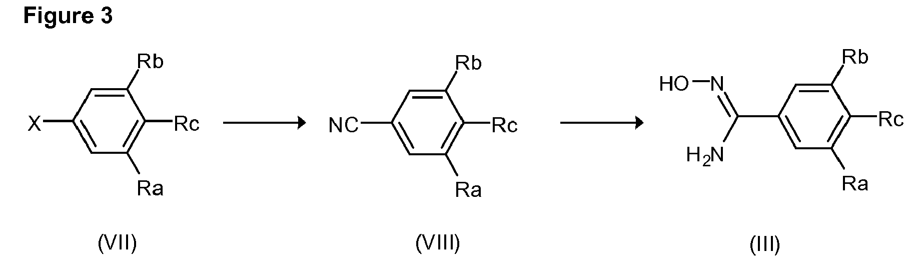

Intermediates of formula (III) may be obtained by the reaction of hydroxylamine hydrochloride or any of its salts with the corresponding nitrile (VIII) in basic media such as sodium bicarbonate or triethylamine in a solvent such as THF, methanol or ethanol and at a temperature from 40 to 100°C. If the cyanoaryl derivative of formula (VIII) is not commercially available it may be obtained from the corresponding derivative of formula (VII) wherein X represents a bromide or a triflate, by reacting with a source of a cyanide such as copper (I) cyanide, in a high boiling point solvent such as N-methylpirrrolidine, dimethylformamide or dimethylsulfoxide at a temperature between 150-200°C. Alternatively dicyanozinc may be used with a palladium catalyst such as tetrakis(triphenylphosphine)-palladium(0) in a high boiling point solvent, in a standard reactor or a microwave reactor.

In the particular case where R_{c} is -(CH₂)₂COOR', intermediates of formula (IIIa) may be obtained following the synthetic path shown in Figure 4.

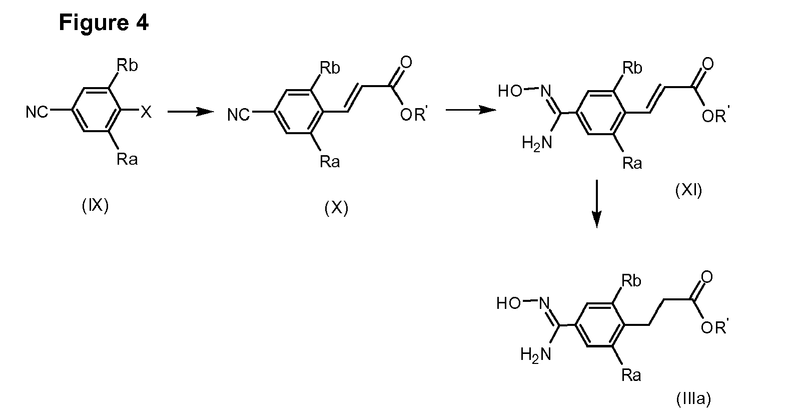

A Heck reaction of the corresponding precursor (IX), wherein X represents triflate, bromo or iodo, which are known or may be prepared according to known procedures, with acrylate yields the intermediate of formula (X). Reaction of these intermediates with hydroxylamine as described above followed by a catalytic hydrogenation, gives intermediates of general formula (IIIa).

In the particular case where R_{c} is -(CH₂)₂-NR'R" group or -(CH₂)₂-NH-(CH₂)(₁₋₃)-NR'R", general compounds of formula (Ib) may be obtained following the synthetic path shown in Figure 5.

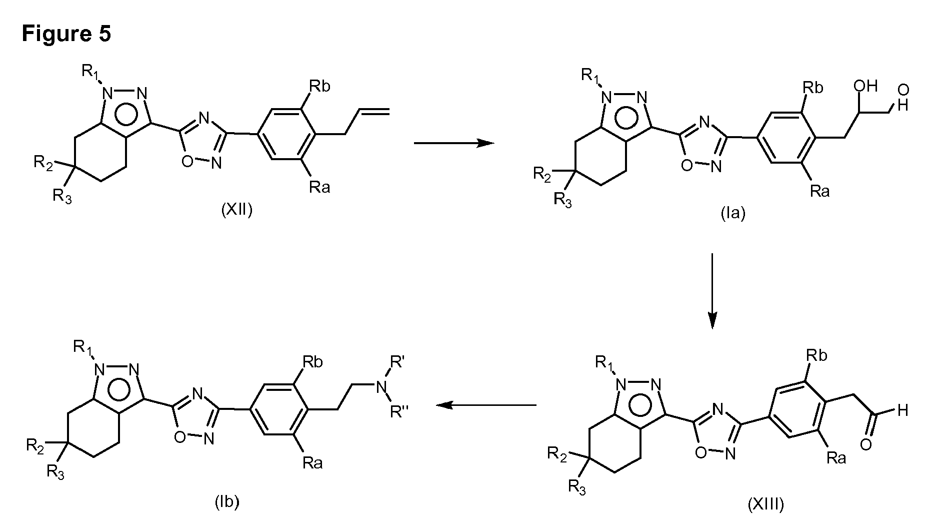

The compounds of general formula (Ib) may be prepared by the reductive amination of the aldehyde derivatives of general formula (XIII) with the corresponding amine or amino acid in acidic media such as acetic acid, in a protic solvent such as methanol or ethanol and with a reductive agent such as sodium borohydride, sodium triacetoxyborohydride or sodium cyanoborohydride at a temperature from 0°C to the boiling point of the solvent.

Intermediates of formula (XIII) may be obtained by oxidation of diols of general formula (Ia) with an oxidative reagent such as sodium periodate, sodium perchlorate, potassium periodate, etc. in a solvent such as methanol, ethanol, THF, dioxane, ether, optionally with the presence of water.

Diol derivatives of general formula (Ia) may be prepared by oxidation of the allyl derivatives of general formula (XII) using a catalytic amount of an oxidazing agent such as osmium tetroxide and a cooxidant such as N-methylmorpholine-N-oxide in a solvent such as methanol, ethanol, THF, dioxane, ether, acetone, optionally with the presence of water.

Allyl derivatives of general formula (XII) may be prepared by the condensation of the corresponding carboximidamide of formula (IIIb) with the corresponding acid formula (II) as described in figure 6.

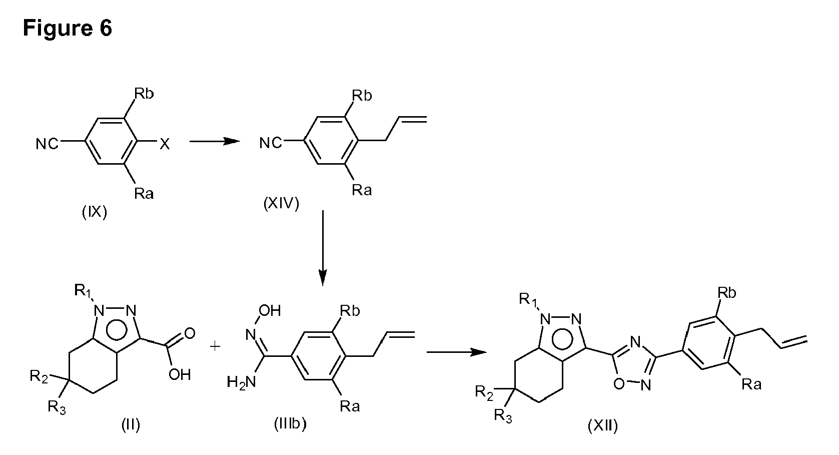

Intermediates of formula (IIIb) may be obtained by standard Stille reaction of the corresponding precursor (IX) wherein X represents triflate, bromo or iodo, using an allylstannane and a catalyst such as tetrakis(triphenylphosphine)-palladium(0), palladium acetate, bis(triphenylphosphine)palladium(II) chloride or tris(dibenzylidene acetone)-dipalladium(0), in a solvent such DMF, acetonitrile, to yield intermediates of general formula (XIV) and subsequent reaction with hydroxylamine as described before.

In the particular case were R_{c} is -O-(CH₂)(₁₋₃)-NR'R" compounds of general formula (Ic) may be obtained as shown in figure 7.

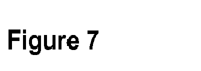

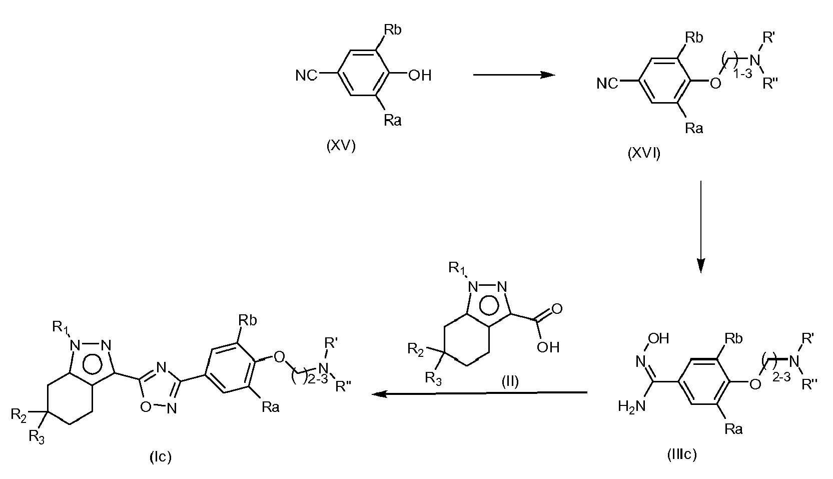

Compounds of formula (Ic) may be prepared by the condensation of the tetrahydroindazole-3-carboxylic acid derivative of formula (II) with the corresponding carboximidamide derivative of formula (IIIc) in a solvent such as dimethylformamide (DMF), N-methylpyrrolidone (NMP), tetrahydrofurane (THF), in the presence of one or more coupling agents such as 2-(1H-benzotriazole-1-yl)-1,2,3,3-tetramethyluronium tetrafluoroborate, N-(3-dimethylaminopropyl)-N'-ethyl-carbodiimide, dicyclohexyl carbodiimide, 1-hydroxybenzotriazole, O-(benzotriazol-1-yl)-N,N,N',N'-tetramethyl uronium hexafluorophosphate, carbonyl diimidazole, and optionally in the presence of a base such as triethylamine, Hünig's base, sodium hydride, potassium carbonate, at a temperature between 40 and 150°C and in a standard reactor.

Compounds of general formula (IIIc) may be obtained by the reaction of hydroxylamine hydrochloride or any of its salts with the corresponding nitrile (XVI) in basic media such as sodium bicarbonate or triethylamine in a solvent such as THF, methanol or ethanol and at a temperature from 40 to 100°C.

The compounds of formula (XVI) may be obtained by the reaction of compounds of general formula (XV) with the corresponding alkylating agent in basic media such as sodium hydride in a solvent such as THF or DMF at a temperature from 0 to 150°C. Alternatively, the phenolic functionality of (XV) may be coupled to suitable alcohol derivatives using a Mitsunobu coupling procedure (Mitsunobu, O., Synthesis 1 (1981)). Preferred coupling conditions include the use of a trialkylphosphine or triarylphosphine, such as tri-n-butylphosphine or triphenylphosphine (PPh₃), in a suitable solvent, such as THF or dichloromethane, and an azodicarbonyl reagent, such as diethyl azodicarboxylate or 1,1'-(azodicarbonyl)dipiperidine.

Compounds of general formula (XV) are commercial available or may be prepared according to known procedures.

In the particular case where R_{c} is -(CH₂)₂-NHC(O)R" group, general compounds of formula (Ie) may be obtained following the synthetic path shown in Figure 8.

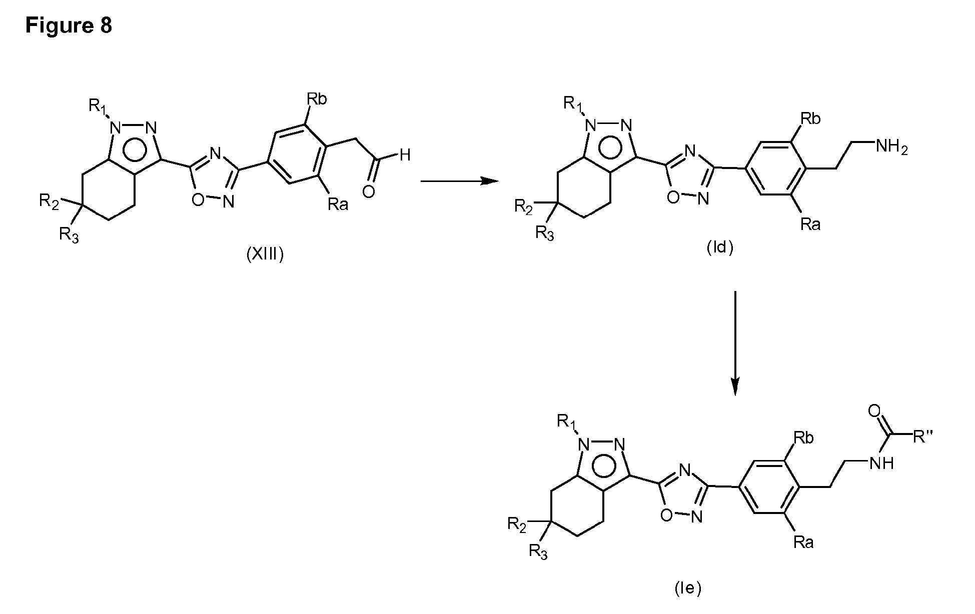

Final compounds of general formula (Ie) may be prepared by the condensation of carboxylic acids with the corresponding amino derivatives of formula (Id) in a solvent such as DMF, NMP or THF, in the presence of one or more coupling agents such as 2-(1H-benzotriazole-1-yl)-1,2,3,3-tetramethyluroniumtetrafluoroborate, N-(3-dimethyl aminopropyl)-N'-ethyl-carbodiimide, dicyclohexylcarbodiimide, 1-hydroxybenzotriazole, O-(benzotriazol-1-yl)-N,N,N',N'-tetramethyluronium hexafluorophosphate, carbonyl diimidazole and optionally in the presence of a base such as triethylamine, Hünig's base, sodium hydride, potassium carbonate, at a temperature between 25 and 80°C and in a standard reactor.

The compounds of general formula (Id) may be prepared by the reductive amination of the aldehyde derivatives of general formula (XIII) with the a source of ammonia such as ammonium acetate or ammonium chloride, in a protic solvent such as methanol or ethanol and with a reductive agent such as sodium borohydride, sodium triacetoxyborohydride or sodium cyanoborohydride at a temperature from 0°C to the boiling point of the solvent.

In the particular case where R_{c} is -(CH₂)₂-NR'R", where R' and R" together with the nitrogen atom to which they are attached form a 5 membered heteroaryl group, which contains, as heteroatom, one N atom and, optionally, one or more further atoms selected from N, and which is optionally substituted with one substituent selected from amino group or carboxy group, compounds of general formula (Ig) may be obtained as shown in figure 9.

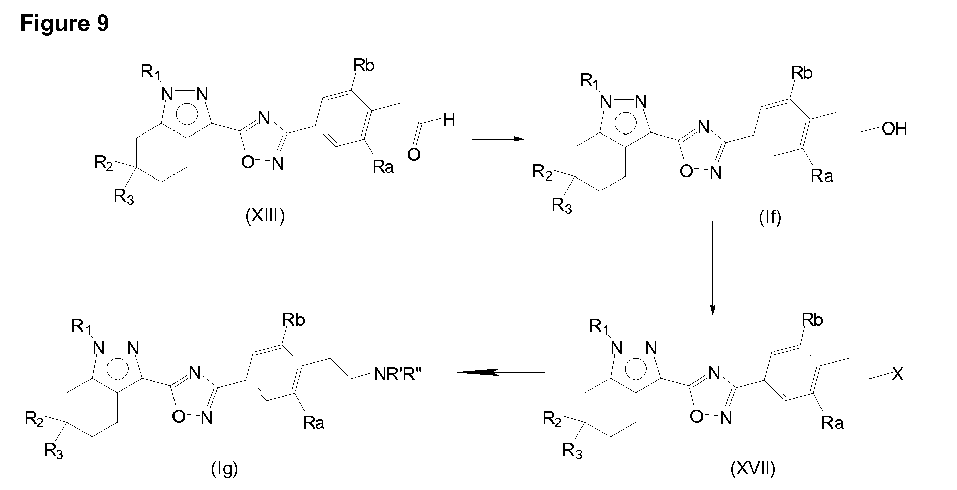

From aldehyde (XIII), the corresponding hydroxy derivative (If) may be prepared by reduction in a solvent such as methanol or dichloroethane, or a mixture of both solvents, with a reductive agent such as sodium borohydride, sodium triacetoxyborohydride or sodium cyanoborohydride at a temperature from 0°C to the boiling point of the solvent.

Intermediates of general formula (XVII), wherein X represents mesylate, tosylate, bromide or iodide, may be prepared by reaction of compounds of formula (If) with methanesulfonyl chloride or p-toluenesulfonyl chloride in a solvent such as dichlorometane, ethyl acetate, DMF or THF in the presence of a base such as triethylamine, pyridine or sodium carbonate at a temperature from 0 to 80°C. An alternative method for the preparation of intermediates (XVII) may be by reaction with halogenating agents such as phosphorus tribromide, HBr in acetic acid, iodine/triphenylphosphine, sodium iodide or tetrabromomethane/triphenylphosphine in a solvent such as dichloromethane, toluene, DMF, acetone at a temperature from 0°C to the boiling point of the solvent.

The compounds of formula (Ig) may be obtained by the reaction of compounds of general formula (XVII) with the corresponding heteroaryl group in a basic media such as sodium hydride or potassium carbonate in a solvent such as THF or DMF at a temperature from 0 to the boiling point of the solvent.

In the particular case where R_{c} is a lineal or branched C₂₋₅ alkyl group substituted with a -P(O)(OH)₂ group, compounds of general formula (Ih) may be obtained as shown in figure 10.

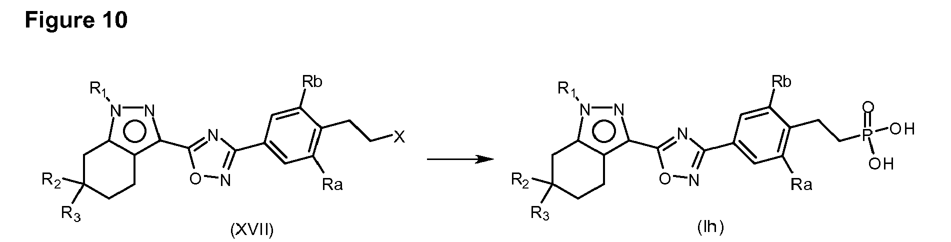

Final compounds of general formula (Ih) may be obtained from intermediates of formula (XVII), wherein X represents mesylate, tosylate, bromide or iodide, with trialkylphosphite at a temperature from 90 to 180°C.

In the particular case where R_{c} represents a lineal or branched C₂₋₅ alkyl group substituted with a triazolyl group, compounds of general formula (Ii) may be obtained as shown in figure 11.

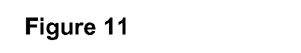

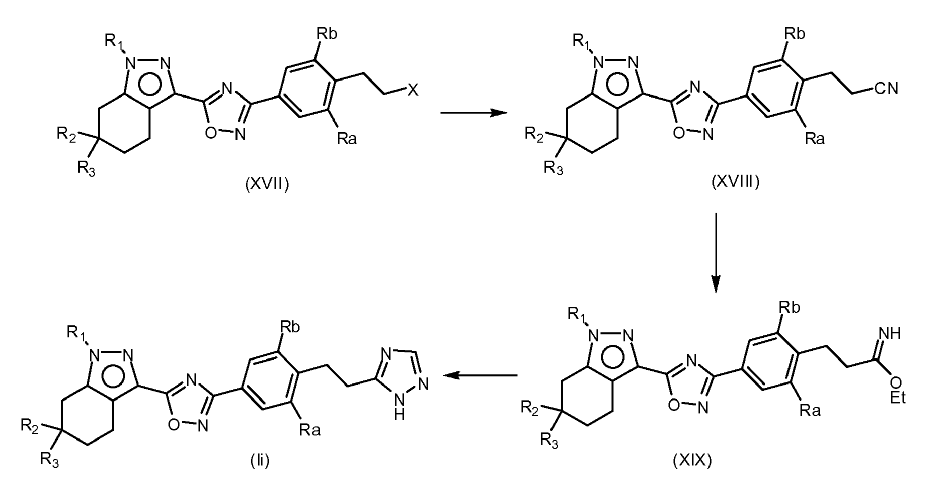

Compounds of general formula (Ii) may be prepared by cyclization of the corresponding imidate intermediates of formula (XIX) with formohydrazide in a solvent such as ethanol, methanol or 2-propanol, optionally in the presence of a base such as triethylamine, pyridine or N-ethyl-N,N-diisopropylamine, at a temperature between 40 and 100°C.

Intermediates of general formula (XIX) may be obtained from the corresponding nitriles of general formula (XVIII) with a saturated solution of HCl in a solvent such as ethanol, at a temperature between 0 and 25°C.

The compounds of formula (XVIII) may be prepared from the intermediates of general formula (XVII) with a cyanide source such as sodium cyanide, potassium cyanide or tetraalkyalmmonium cyanide, in a solvent such as DMF or dimethylsulfoxide and at a temperature between 40 and 120°C.

Starting compounds are commercially available or may be obtained following the conventional synthetic methods already known in the art.

Depending on the nature of the functionalities present in the residues Rₐ to R_{c}, these functionalities may require temporary protection. Appropriate protecting groups are known in the art and include e.g a *tert*-butyl or ethyl or methyl to protect an acid, a *tert-*butyloxycarbonyl (BOC) to protect an amine, etc. These protecting groups may be employed according to standard methodology (e.g. T.W. Greene, P. G. M. Wuts, Protective Groups in Organic Synthesis, 3rd Edition, Wiley New York, 1991).

The syntheses of the compounds of the invention are illustrated by the following Examples (1 to 24) including Preparation Examples (1 to 37) which do not limit the scope of the invention in any way.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B, C and D. The injection volume was 5 microliter for method A, B and D and 3 microliter for method C. Diode array chromatograms were collected at 210 nM.

| | 0% B | 0 to 95% B | 95% B |
|---|---|---|---|
| A | 0.2 min | 3 min | 0.8 min |
| B | 0.5 min | 6.5 min | 1 min |
| C | 0 min | 20 min | 4 min |
| D | 0 min | 10.5 min | 1.5 min |

### General Purification Method:

The solid was dissolved in DMSO/MeOH, injected into a Biotage C18 silica column (40M, 25M or 25S according to the crude amount) and eluted on the SP1® automated purification system from Biotage. The gradient used was H2O/Acetonitrile/MeOH (1:1) (0.1% v/v HCOONH4 both phases) from 0% to 100% acetonitrile/MeOH (1:1) in 80 column volumes. The appropriate fractions were collected and the organic solvent evaporated under reduced pressure or liofilized.

### GENERAL SYNTHETIC METHODS:

### General Method 1:

A mixture of the corresponding benzonitrile (39.2 mmol) in methanol (50 mL), hydroxylamine hydrochloride (5.45 g, 78.4 mmol) and sodium bicarbonate (13.17 g, 156.8 mmol) was stirred at 75°C for 6h. The mixture was filtered off and the filtrate evaporated to dryness to yield the title compound (75-100% yield) as a white solid.

### General Method 2:

A mixture of the corresponding acid derivative (1.13 mmol), EDC (1.13 mmol) and HOBt (1.13 mmol) in DMF (5 mL) was stirred at room temperature for 10 min. Then the corresponding benzimidamide (1.13 mmol) was added and the mixture stirred at 140°C for 4h. The reaction mixture was poured over basic ice/water and the solid formed filtered and washed with water, dried in a vacuum oven to give the desired compound (yield=10-90%) as a white solid.

### General Method 3:

To a mixture of the corresponding methyl or ethyl ester (0.67 mmol) in methanol or ethanol (3 mL) respectively a 2M solution of aqueous NaOH (12 mmol) was added and the reaction mixture stirred at 90°C overnight. The organic solvent was evaporated, water was added and the mixture extracted with diethyl ether. The pH of the aqueous layer was adjusted to 5-6 and the solid formed filtered and dried in the vacuum oven. The desired compound (60-95% yield) was obtained as a white solid.

### General Method 4:

A mixture of the corresponding *tert-*butyl ester (0.56 mmol) in 4M HCl in dioxane (3.5 mL) was stirred at r.t. overnight. The solvent was evaporated and the solid obtained washed with diisopropyl ether twice. The solid was dried in the vacuum oven to yield (70-95% yield) of the desired compound.

### General Method 5:

A mixture of the corresponding acid (0.46 mmol), EDC (133 mg, 0.69 mmol), HOBt (94mg, 0.69 mmol) and 32% solution of aqueous ammonia (85 µl, 0.69 mmol) in DMF (6.5 mL) was stirred overnight at room temperature. Then ethyl acetate and water were added and the organic layer separated, washed with 4% NaHCO₃, water and brine and dried to give the title compound (5-77% yield) as a white solid.

### General Method 6:

To a solution of the corresponding aldhehyde (200 mg, 0,46 mmol) in methanol (10ml) the corresponding amine (44 µl, 0,55 mmol) and acetic acid (236 µl, 4,14 mmol) were added and the mixture was stirred at room temperature for 30 min. Then NaBH₃CN (15 mg, 0.23 mmol) was added and the reaction mixture stirred at r.t. overnight. Solvent was concentrated and the residue dissolved in ethyl acetate, washed with water and brine, dried over magnesium sulphate, filtered and concentrated. The crude obtained was purified according to the General Purification Method to give the desired compound (25-85% yield).

When the defined groups R¹ to R³ and Ra to Rb are susceptible to chemical reaction under the conditions of the hereinbefore described processes or are incompatible with said processes, conventional protecting groups may be used in accordance with standard practice, for example see T.W. Greene and P.G.M. Wuts in "protective Groups in Organic Chemistry", 3rd Edition, John Wiley&Sons (1999). It may be that deprotection will form the last step in the synthesis of compounds of formula (I).

The syntheses of the compounds of the invention and their intermediates are illustrated by the following Examples (1 to 24) including Preparation Examples (1 to 37) which do not limit the scope of the invention in any way Starting compounds are commercially available or may be obtained following the conventional synthetic method already known in the art.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B and C. The injection volume was 5 microliter for method A and B and 3 microliter for method C. Diode array chromatograms were collected at 210 nM.

| Method | 0% B | 0 to 95% B | 95% B |
|---|---|---|---|
| A | 0.2 min | 3min | 0.8min |
| B | 0.5min | 6.5min | 1 min |
| C | 0min | 20min | 4min |

### PREPARATION EXAMPLES

### Preparation 1

### Ethyl 2-(4,4-dimethyl-2-oxocyclohexyl)-2-oxoacetate

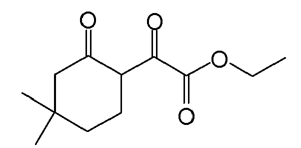

Sodium (8.47 g, 0.37 mol) was slowly added to ethanol (500 mL), then 3,3-dimethylcyclohexanone (15.5 g, 0.12 mol) in ethanol (200 mL) was added and the mixture stirred at room temperature for 15 min. Finally diethyl oxalate (16.65 mL, 0.12 mol) in ethanol (100 mL) was added and the mixture stirred overnight at r.t. The solvent was concentrated and the crude redissolved in water and dichloromethane. A 5N solution of HCl was added until acid pH and the layers separated. The aqueous layer was extracted with dichloromethane and the combined organic layer was washed with brine, dried over sodium sulphate and concentrated. An oil that contained the desired compound and the desired compound in the acid form was obtained. The mixture was used for the next reaction without further purification (69% yield).
LRMS: m/z 227 (M+1)⁺
Retention time: 6.50 min (Method B)

### Preparation 2

### Ethyl 1-ethyl-6,6-dimethyl-4,5,6,7-tetrahydro-1H-indazole-3-carboxylate

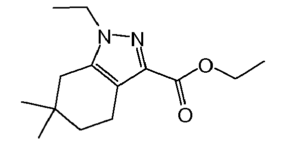

To a suspension of ethylhydrazine oxalic acid (17. 8 g, 120 mmol) in ethanol (200 mL) triethylamine (18 mL, 130 mmol) was added and solution was poured onto Preparation 1 (19.2 g, 80 mmol) in ethanol (300 mL). The reaction mixture was stirred at r.t. for 4 h and then the solvent concentrated. The residue was redissolved in ethyl acetate/water and the layers separated. The aqueous layer was extracted with ethyl acetate and the combined organic layers were washed with brine, dried over sodium sulphate and concentrated. The mixture was used for the next reaction without further purification (82% yield).
LRMS: m/z 251 (M+1)⁺
Retention time: 6.59 min (Method B)

### Preparation 3

### 1-ethyl-6,6-dimethyl-4,5,6,7-tetrahydro-1H-indazole-3-carboxylic acid

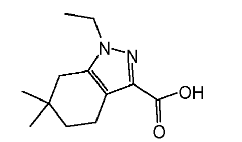

Obtained (86%) from Preparation 2 following General Method 3.
LRMS: m/z 223 (M+1)⁺
Retention time: 5.66 min (Method B)
¹H NMR (200 MHz, DMSO-D6) δ ppm 1.0 (s, 6 H) 1.2 (t, *J*=7.2 Hz, 2 H) 1.4 (t, *J*=6.2 Hz, 2 H) 2.3 (s, 2 H) 2.6 (t, *J*=6.2 Hz, 2 H) 4.0 (q, *J*=7.0 Hz, 2 H).

### Preparation 4

### 3-cyclopropyl-4-hydroxybenzonitrile

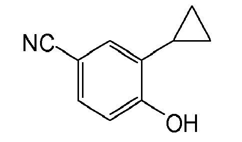

To a solution of 3-bromo-4-hydroxybenzonitrile (5 g, 25.25 mmol) in toluene/water (20/0.5 mL) cyclopropylboronic acid (2.39 g, 27.77 mmol), tricyclohexylphosphine (2.83 g, 10.1 mmol), potassium phosphate (16.08 g, 75.75 mmol) and palladium acetate (0.57 g, 2.52 mmol) were added under argon atmosphere and mixture stirred at 100°C overnight. Crude was purified according to General Purification Method to yield the title compound (50% yield).
LRMS: m/z 158 (M-H)⁺
Retention time: 5.22 min (Method B)

### Preparation 5

### 4-cyano-2-cyclopropylphenyl trifluoromethanesulfonate

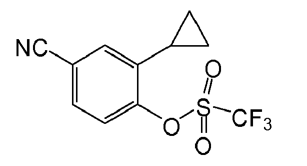

To a mixture of dichloromethane (10 mL) and pyridine (0.53 mL, 6.51 mmol) at 0°C trifluoromethanesulfonic anhydride (1.68 mL, 5.96 mmol) was added and mixture stirred at this temperature for 10 min. Then Preparation 4 (0.86 g, 5.42 mmol) dissolved in dichloromethane (5 mL) was added and mixture stirred at r.t. for 2 h. Reaction was further diluted with dichloromethane and washed with water and brine, organic layer was dried over magnesium sulphate, filtered and concentrated. Crude thus obtained was purified by normal phase chromatography with isocratic dichloromethane to yield the title compound (85% yield).
LRMS: No signal
Retention time: 6.80 min (Method B)

### Preparation 6

### Ethyl 3-(4-cyano-2-cyclopropylphenyl)acrylate

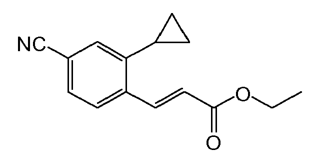

To a mixture of Preparation 5 (1.1 g, 3.78 mmol), ethyl acrylate (0.75 g, 7.55 mmol), 1,1'-Bis(diphenylphosphino)ferrocene (77.9 mg, 0.19 mmol) and triethylamine (1.05 mL, 7.55 mmol) in DMF (8 mL), palladium acetate (42.4 mg, 0.19 mmol) was added under nitrogen atmosphere. The reaction mixture was stirred overnight at 110°C. After cooling to room temperature, the mixture was poured onto water and extracted with diethyl ether twice. The combined organic layer was washed with water and brine, dried over magnesium sulphate and concentrated. Crude thus obtained was purified by normal phase chromatography using hexane and ethyl acetate 8:2 to yield the title compound (61 % yield).
LRMS: no signal
Retention time: 6.58 min (Method B)
¹H NMR (300 MHz, CHLOROFORM-*d*) δ ppm 0.71-0.77 (m, 2H), 1.10-1.15 (m, 2H), 1.34-1.39 (t, 3H), 2.02-2.08 (m, 1H), 4.27-4.34 (q, 2H), 6.43-6.49 (d, 1H), 7.37 (s, 1H), 7.51 (s, 1H), 7.59-7.62 (d, 1H), 6.23-6.28 (d, 1H).

### Preparation 7

### Ethyl 3-(2-cyclopropyl-4-(N'-hydroxycarbamimidoyl)phenyl)acrylate

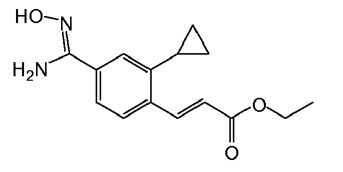

Obtained from Preparation 6 following General Method 1 (90% yield).
LRMS: m/z 275 (M+H)⁺
Retention time: 4.47 min (Method B)

### Preparation 8

### Ethyl 3-(2-cyclopropyl-4-(N'-hydroxycarbamimidoyl)phenyl)propanoate

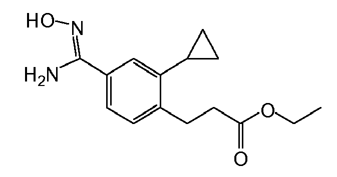

Preparation 7 (0.70 g, 2.55 mmol) was dissolved in methanol (10 mL) and sodium acetate (0.314 g, 3.83 mmol) was added. Finally palladium chloride (90.5 mg, 0.51 mmol) was added and the mixture hydrogenated for 5 h. The catalyst was filtered off and the filtrate concentrated. The residue was redissolved in dichloromethane and washed with water. The organic layer was dried over magnesium sulphate and concentrated to yield the title compound (63% yield).
LRMS: m/z 277 (M+H)⁺
Retention time: 4.07 min (Method B)

### Preparation 9

### Ethyl 3-(2-cyclopropyl-4-(5-(1-ethyl-6,6-dimethyl-4,5,6,7-tetrahydro-1 H-indazol-3-yl)-1,2,4-oxadiazol-3-yl)phenyl)propanoate

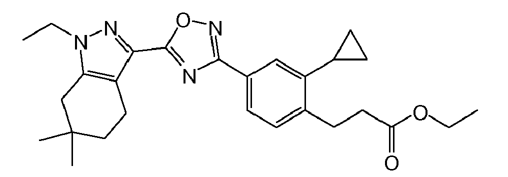

Obtained (40%) from Preparation 3 and Preparation 8 following General Method 2.
LRMS: m/z 463 (M+H)⁺
Retention time: 7.93 min (Method B)

### Preparation 10

### Ethyl 1,6,6-trimethyl-4,5,6,7-tetrahydro-1H-indazole-3-carboxylate

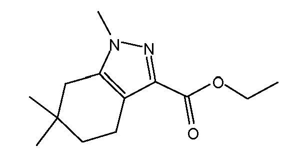

Obtained (71 % yield) from Preparation 1 and methyl hydrazine following the experimental procedure described for Preparation 2.
LRMS: m/z 237 (M+1 )⁺
Retention time: 6.39 min (Method B)

### Preparation 11

### 1,6,6-trimethyl-4,5,6,7-tetrahydro-1H-indazole-3-carboxylic acid

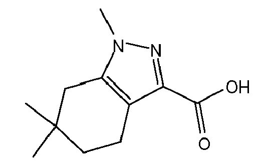

Obtained (95% yield) from Preparation 10 following General Method 3.
LRMS: m/z 209 (M+1)⁺
Retention time: 5.35 min (Method B)

### Preparation 12

### 4-cyano-2,6-dimethylphenyl trifluoromethanesulfonate

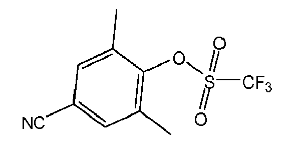

To a suspension of 4-hydroxy-3,5-dimethylbenzonitrile (5.10 g, 34.65 mmol) in dichloromethane (100 mL) triethylamine (7.25 mL, 52.02 mmol) was added. To the solution obtained 1,1,1-trifluoro-N-phenyl-N-(trifluoromethylsulfonyl)methansulfonamide (14.9 g, 41.7 mmol) was added and the mixture stirred overnight at r.t. More dichloromethane was added and then washed with 0.5N NaOH, water and brine. The organic layer was dried over magnesium sulphate and concentrated to yield the desired compound as a solid (100% yield).
LRMS: no signal
Retention time: 6.90 min (Method B)

### Preparation 13

### 4-allyl-3,5-dimethylbenzonitrile

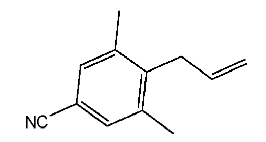

To a solution of Preparation 12 (5 g, 14.74 mmol) in DMF (175 mL) under nitrogen atmosphere, allyltributylstannane (5.50 mL, 17.74 mmol) and Pd(PPh₃)₄ (1.71 g, 1.48 mmol) were added and the mixture stirred overnight at 90°C. The reaction mixture was poured onto ice/water and ethyl acetate was added. The mixture was filtered over Decalite and the organic layer separated, washed with water and brine, dried over magnesium sulphate and concentrated. The crude obtained was used without further purification (80% yield).
LRMS: no signal
Retention time: 6.69 min (Method B)

### Preparation 14

### 4-allyl-N'-hydroxy-3,5-dimethylbenzimidamide

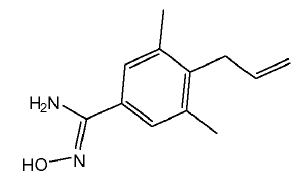

Obtained (69% yield) from Preparation 13 following General Method 1.
LRMS: m/z 205 (M+H)⁺
Retention time: 4.07 min (Method B)

### Preparation 15

### 3-(4-allyl-3,5-dimethylphenyl)-5-(1,6,6-trimethyl-4,5,6,7-tetrahydro-1H-indazol-3-yl)-1,2,4-oxadiazole

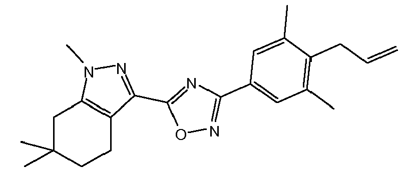

Obtained (33% yield) from Preparation 11 and Preparation 14 following General Method 2.
LRMS: m/z 377 (M+H)⁺
Retention time: 8.08 min (Method B)

### Preparation 16

### 3-(2,6-dimethyl-4-(5-(1,6,6-trimethyl-4,5,6,7-tetrahydro-1H-indazol-3-yl)-1,2,4-oxadiazol-3-yl)phenyl)propane-1,2-diol

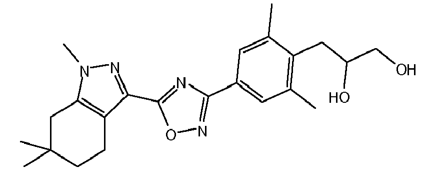

To a solution of Preparation 15 (4.1 g, 10,84 mmol) in a mixture of THF/*tert*-butanol (90 mL / 14 mL) 4-methylmorpholine 4-oxide (2.54 g, 21,68 mmol) and osmium(VIII) oxide (1.32 mL, 0,22 mmol) were added. The reaction mixture was stirred overnight at r.t. Then 40% solution of Na₂SO₃ was added and the mixture stirred for 30 min. Ethyl acetate was added and the organic layer separated, washed twice with water, dried over magnesium sulphate and concentrated to give the title compound (92% yield).
LRMS: m/z 411 (M+1)⁺
Retention time: 17.80 min (Method C)

### Preparation 17

### 2-(2,6-dimethyl-4-(5-(1,6,6-trimethyl-4,5,6,7-tetrahydro-1H-indazol-3-yl)-1,2,4-oxadiazol-3-yl)phenyl)acetaldehyde

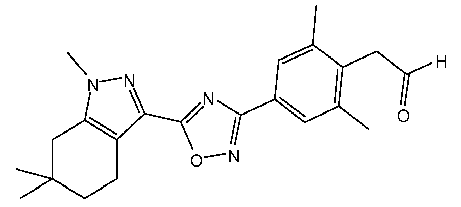

To a suspension of Preparation 16 (4 g, 10 mmol) in methanol (108 mL) and water (12 mL) NalO₄ (3.2 g, 10 mmol) was added and the mixture stirred overnight at r.t. Methanol was concentrated and the residue dissolved in ethyl acetate and water. Organic layer was separated, washed with water and brine, dried over magnesium sulphate and concentrated to give the title compound (97% yield).
LRMS: m/z 379 (M+H)⁺
Retention time: 7.33 min (Method B)

### Preparation 18

### 2-(2,6-dimethyl-4-(5-(1,6,6-trimethyl-4,5,6,7-tetrahydro-1H-indazol-3-yl)-1,2,4-oxadiazol-3-yl)phenyl)ethanamine

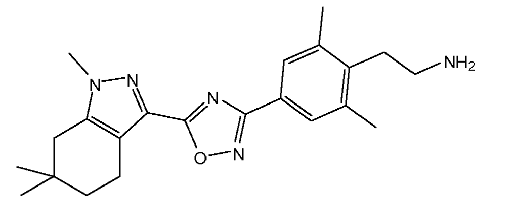

Obtained (27%) as hydrochloride salt from Preparation 17 following General Method 6, using ammonium acetate and sodium triacetoxyborohydride as reducing agent.
LRMS: m/z 380 (M+H)⁺
Retention time: 5.57 min (Method B)
¹H NMR (300 MHz, DMSO-*d*₆) δ ppm 1.02 (s, 6 H) 1.55 (t, *J*=5.22 Hz, 2 H) 2.30 - 2.57 (m, 8 H) 2.67 - 2.94 (m, 4 H) 2.90 - 3.14 (m, 2 H) 3.83 (s, 3 H) 7.74 (s, 2 H) 8.12 (br. s., 3 H).

### Preparation 19

### tert-Butyl 2-(2,6-dimethyl-4-(5-(1,6,6-trimethyl-4,5,6,7-tetrahydro-1H-indazol-3-yl)-1,2,4-oxadiazol-3-yl)phenethylamino)-2-oxoethylcarbamate

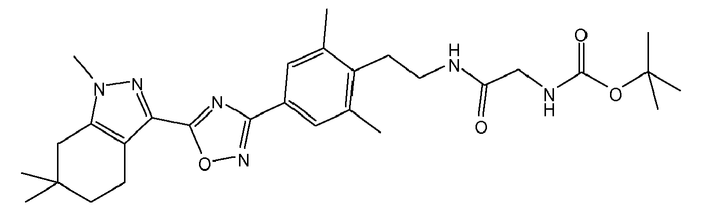

Obtained (44%) from Preparation 18 and 2-(*tert*-butoxycarbonylamino)acetic acid following General Method 5.
LRMS: m/z 537 (M+H)⁺
Retention time: 7.48 min (Method B)
¹H NMR (300 MHz, CHLOROFORM-d) δ ppm 1.07 (s, 6 H) 1.46 (s, 9 H) 1.53 - 1.67 (m, 5 H) 2.40 (s, 2 H) 2.43 (s, 6 H) 2.77 - 3.05 (m, 4 H) 3.32 - 3.57 (m, 2 H) 3.81 (d, *J*=5.77 Hz, 2 H) 3.87 (s, 3 H) 7.89 (s, 2 H).

### Preparation 20

### tert-Butyl 1-(2,6-dimethyl-4-(5-(1,6,6-trimethyl-4,5,6,7-tetrahydro-1H-indazol-3-yl)-1,2,4-oxadiazol-3-yl)phenethylamino)-2-methyl-1-oxopropan-2-ylcarbamate

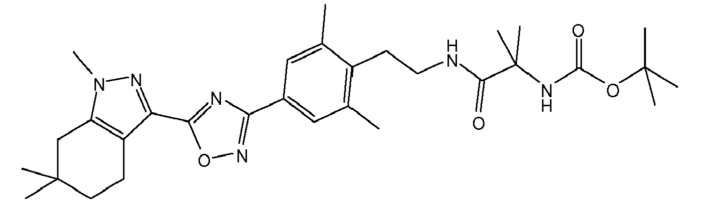

Obtained (46%) from Preparation 18 and 2-(*tert*-butoxycarbonylamino)-2-methylpropanoic acid following General Method 5.
LRMS: m/z 565 (M+H)⁺
Retention time: 7.63 min (Method B)
¹H NMR (300 MHz, CHLOROFORM-*d*) δ ppm 1.08 (s, 6 H) 1.45 (s, 9 H) 1.51 (s, 6 H) 1.62 (t, *J*=6.32 Hz, 2 H) 2.40 (s, 2 H) 2.46 (s, 6 H) 2.78 - 3.02 (m, 4 H) 3.14 - 3.49 (m, 2 H) 3.87 (s, 3 H) 7.89 (s, 2 H).

### Preparation 21

### Methyl 2-(2-(tert-butoxycarbonylamino)ethoxy)-5-cyanobenzoate

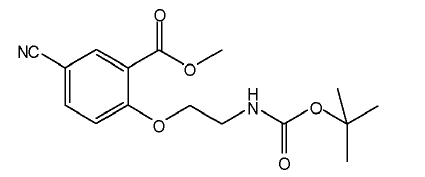

To a suspension of methyl 5-cyano-2-hydroxybenzoate (2 g, 11.29 mmol) in THF (20 mL) *tert*-butyl-2-hydroxycarbamate (2.1 mL, 13.55 mmol) and triphenylphosphine (8.88 g, 33.87 mmol) were added. Mixture was cooled to 0°C and DEAD (6.9 mL, 33.87 mmol) was added and mixture stirred at r.t. for 24 h. Solvent was removed and crude purified by normal phase chromatography with hexane/ethyl acetate from 20 to 40% to yield the title compound as a white solid (100% yield).
¹H NMR (250 MHz, DMSO-D6) δ ppm 1.37 (s, 9 H) 3.32 (t, *J*=5.6 Hz, 2 H) 3.81 (s, 3 H) 4.14 (t, *J*=5.5 Hz, 2 H) 6.99 (t, *J*=5.2 Hz, 1H) 7.34 (d, *J*=8.8 Hz, 1H) 7.99 (dd, *J*=8.8, 2.2 Hz, 1H) 8.05 (d, *J*=2.2 Hz, 1H).

### Preparation 22

### Methyl 2-(2-(tert-butoxycarbonylamino)ethoxy)-5-(N'-hydroxycarbamimidoyl) benzoate

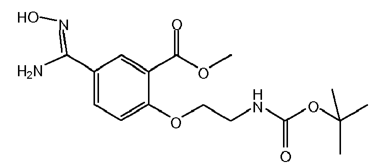

Obtained (100%) from Preparation 21 following General Method 1.
¹H NMR (250 MHz, DMSO-D6) δ ppm 1.44 (s, 9 H) 3.29 (m, 2 H) 3.91 (s, 3 H) 3.85 (s, 3 H) 4.16 (t, *J*=5.2 Hz, 2 H) 6.97 (t, *J*=5.5 Hz, 1H) 7.26 (d, *J*=8.2 Hz, 1 H) 7.76 (d, *J*=8.5 Hz, 1H) 8.07 (sa, 1H).

### Preparation 23

### Methyl 2-(2-(tert-butoxycarbonylamino)ethoxy)-5-(5-(1,6,6-trimethyl-4,5,6,7-tetrahydro-1H-indazol-3-yl)-1,2,4-oxadiazol-3-yl)benzoate

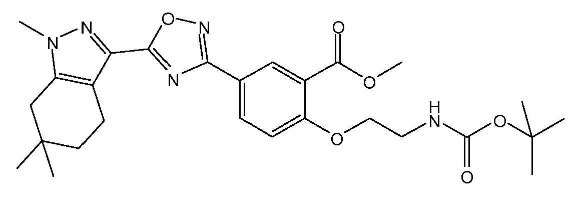

Obtained (31 %) from Preparation 11 and Preparation 22 following General Method 2.
¹H NMR (250 MHz, DMSO-D6) δ ppm 1.01 (s, 6 H) 1.38 (s, 9 H) 1.55 (t, *J*=6.8 Hz, 2 H) 2.47 (s, 2 H) 2.79 (t, *J*=5.4 Hz, 2 H) 3.32 (m, 2 H) 3.82 (s, 3 H) 3.85 (s, 3 H) 4.15 (t, *J*=5.5 Hz, 2 H) 6.91 (t, *J*=5.5 Hz, 1 H) 7.34 (d, *J*=8.7 Hz, 1 ) 8.16 (dd, *J*=8.1, 2.2 Hz, 1 H) 8.32 (sa, 1 H).

### Preparation 24

### 2-(2-(tert-butoxycarbonylamino)ethoxy)-5-(5-(1,6,6-trimethyl-4,5,6,7-tetrahydro-1H-indazol-3-yl)-1,2,4-oxadiazol-3-yl)benzoic acid

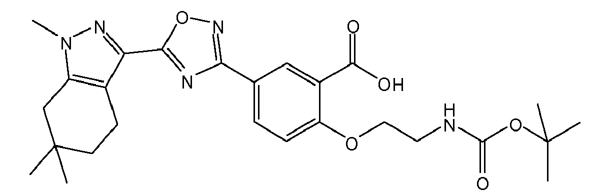

Obtained (91 %) from Preparation 23 following General Method 3.
¹H NMR (250 MHz, DMSO-D6) δ ppm 1.01 (s, 6 H) 1.38 (s, 9 H) 1.54 (t, *J*=5.8 Hz, 2 H) 2.47 (s, 2 H) 2.79 (t, *J*=5.4 Hz, 2 H) 3.32 (m, 2 H) 3.82 (sa, 3 H) 4.15 (t, *J*=5.4 Hz, 2 H) 6.92 (sa, 1 H) 7.35 (d, *J*=8.6 Hz, 1 H) 8.16 (d, *J*=8.1 Hz, 1 H) 8.32 (sa, 1 H).

### Preparation 25

### 4-(4-hydroxypiperidin-1-yl)benzonitrile

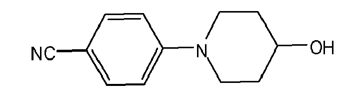

4-fluorobenzonitrile (500 mg, 4.13 mmol), piperidin-4-ol (418 mg, 4.13 mmol) and potassium carbonate (626 mg, 4.53 mmol) were dissolved in DMSO (9 mL) and mixture stirred at 120°C for 75 min. Solution was poured onto water and solid obtained was filtered, washed with water and dried in the vacuum oven to yield the title compound (73% yield).
LRMS: m/z 203 (M+1)⁺
Retention time: 4.75 min (Method C)
¹H NMR (300 MHz, CHLOROFORM-*d*) δ ppm 1.47 - 1.78 (m, 2 H) 1.83 - 2.15 (m, 2 H) 2.92 - 3.25 (m, 2 H) 3.58 - 3.81 (m, 2 H) 3.84 - 4.11 (m, 1 H) 6.87 (d, *J*=9.06 Hz, 2 H) 7.48 (d, *J*=8.79 Hz, 2 H).

### Preparation 26

### N'-hydroxy-4-(4-hydroxypiperidin-1-yl)benzimidamide

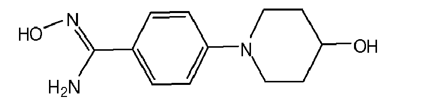

Obtained (31%) from Preparation 25 following General Method 1.
LRMS: m/z 236 (M+1)⁺
Retention time: 2.82 min (Method B)

### Preparation 27

### 2-(2,6-dimethyl-4-(5-(1,6,6-trimethyl-4,5,6,7-tetrahydro-1H-indazol-3-yl)-1,2,4-oxadiazol-3-yl)phenyl)etanol

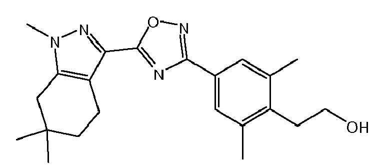

To a solution of Preparation 17 (1.5 g, 3.96 mmol) in methanol/dichloroethane (15/5 mL) NaBH₄ (0.45 g, 11.9 mmol) was added and mixture stirred overnight at r.t. Then solvent was removed, crude redissolved in dichloromethane and washed with water, organic layer dried and concentrated to yield the title compound as a white solid (97% yield).
LRMS: m/z 381 (M+1 )⁺
Retention time: 18.74 min (Method C)
¹H NMR (300 MHz, CHLOROFORM-D) δ ppm 1.1 (s, 6 H) 1.6 (t, 2 H) 2.4 (s, 2 H) 2.4 (s, 6 H) 2.9 (t, *J*=6.0 Hz, 2 H) 3.0 (t, *J*=7.4 Hz, 2 H) 3.8 (m, *J*=6.0, 6.0, 6.0 Hz,2H)3.9(s,3H)7.9(s,2H).

### Preparation 28

### 2,6-dimethyl-4-(5-(1,6,6-trimethyl-4,5,6,7-tetrahydro-1H-indazol-3-yl)-1,2,4-oxadiazol-3-yl)phenethyl methanesulfonate

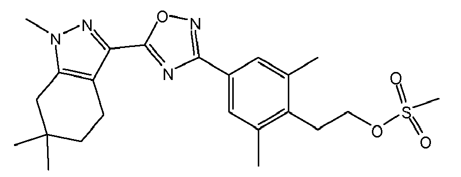

To a suspension of Preparation 27 (1 g, 2.65 mmol) in dichloromethane (20 mL) triethylamine (1.1 mL, 7.9 mmol) was added, mixture cooled to 0°C and methanesulfonyl chloride (0.47 mL, 5.9 mmol) was added dropwise. Reaction was stirred at r.t. for 5 h and then poured onto saturated NaHCO₃ solution on ice, organic layer was washed with brine, dried over magnesium sulphate, filtered and concentrated. The title compound was obtained without any further purification (85% yield).
LRMS: m/z 459 (M+1)⁺
Retention time: 7.45 min (Method B)
¹H NMR (300 MHz, DMSO-*d*₆) δ ppm 1.02 (s, 6 H) 1.55 (t, *J*=6.32 Hz, 2 H) 2.43 (s, 6 H) 2.47 (s, 2 H) 2.80 (t, *J*=6.18 Hz, 2 H) 3.06 - 3.23 (m, 5 H) 3.83 (s, 3 H) 4.33 (t, *J*=7.42 Hz, 2 H) 7.75 (s, 2 H).

### Preparation 29

### Ethyl 3-(4-cyano-2,6-dimethylphenyl)acrylate

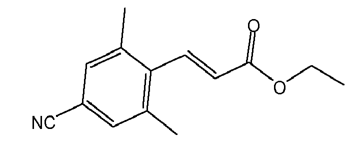

Obtained (59% yield) from Preparation 12 and ethyl acrylate following the procedure described in Preparation 6.
LRMS: m/z 230
Retention time: 6.50 min (Method B)

### Preparation 30

### Ethyl 3-(4-(N'-hydroxycarbamimidoyl)-2,6-dimethylphenyl)acrylate

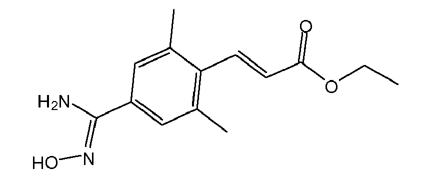

Obtained (66%) from Preparation 29 following General Method 1.
LRMS: m/z 263 (M+1)⁺
Retention time: 4.20 min (Method D)

### Preparation 31

### Ethyl 3-(4-(N'-hydroxycarbamimidoyl)-2,6-dimethylphenyl)propanoate

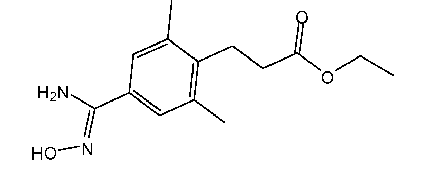

Obtained from Preparation 30 following the procedure described in Preparation 8 (77% yield).
LRMS: m/z 265 (M+1)⁺
Retention time: 4.13 min (Method D)
¹H NMR (300 MHz, CHLOROFORM-*d*) δ ppm 1.28 (t, *J*=7.14 Hz, 3 H) 2.35 (s, 6 H) 2.38 - 2.50 (m, 2 H) 2.84 - 3.12 (m, 2 H) 4.17 (q, *J*=7.14 Hz, 2 H) 4.85 (br. s., 2 H) 7.27 (s, 2 H).

### Preparation 32

### Ethyl 3-(2,6-dimethyl-4-(5-(1,6,6-trimethyl-4,5,6,7-tetrahydro-1H-indazol-3-yl)-1,2,4-oxadiazol-3-yl)phenyl)propanoate

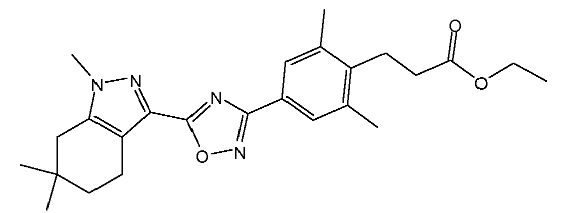

Obtained (54%) from Preparation 11 and Preparation 31 following General Method 2.
LRMS: m/z 437 (M+1)⁺
Retention time: 7.87 min (Method B)
¹H NMR (300 MHz, CHLOROFORM-*d*) δ ppm 1.07 (s, 6 H) 1.28 (t, *J*=7.14 Hz, 3 H) 1.60 (t, *J*=6.45 Hz, 2 H) 2.40 (m, 8 H) 2.42 - 2.52 (m, 2 H) 2.90 (t, *J*=6.32 Hz, 2 H) 2.96 - 3.11 (m, 2 H) 3.86 (s, 3 H) 4.18 (q, *J*=7.14 Hz, 2 H) 7.88 (s, 2 H).

### Preparation 33

### 3-(2,6-dimethyl-4-(5-(1,6,6-trimethyl-4,5,6,7-tetrahydro-1H-indazol-3-yl)-1,2,4-oxadiazol-3-yl)phenyl)propanenitrile

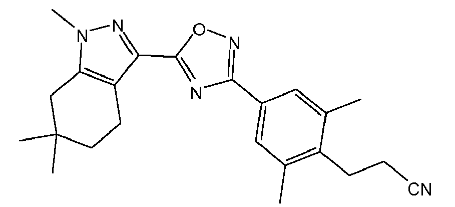

To a solution of Preparation 28 (150 mg, 0.39 mmol) in DMF (3 mL) sodium cyanide was added and mixture stirred overnight at 60°C. Crude was diluted with ethyl acetate and washed with saturated NaHCO₃ and brine, dried over magnesium sulphate, filtered and concentrated. Desired product was obtained without further purification (100% yield).
LRMS: m/z 390 (M+1)⁺
Retention time: 7.40 min (Method B)

### Preparation 34

### Ethyl 3-(2,6-dimethyl-4-(5-(1,6,6-trimethyl-4,5,6,7-tetrahydro-1H-indazol-3-yl)-1,2,4-oxadiazol-3-yl)phenyl)propanimidate

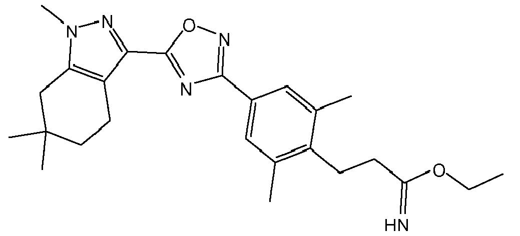

To Preparation 33 (150 mg, 0.39 mmol) saturated HCl solution in ethanol (8 mL) was added at 0°C and mixture stirred at this temperature for 3 h. Solvent was removed and product obtained without further purification (100% yield).
LRMS: m/z 436 (M+1)⁺
Retention time: 6.07 min (Method B)

### Preparation 35

### Ethyl 1-(2,6-dimethy)-4-(5-(1,6,6-trimethy)-4,5,6,7-tetrahydro-1H-indazol-3-yl)-1,2,4-oxadiazol-3-yl)phenethyl)-4-fluoropiperidine-4-carboxylate

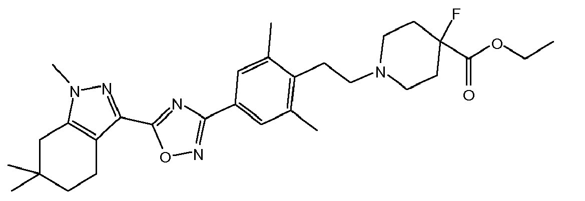

Obtained (74%) from Preparation 17 and ethyl 4-fluoropiperidine-4-carboxylate following General Method 6.
LRMS: m/z 538 (M+1)⁺
Retention time: 5.92 min (Method B)

### Preparation 36

### 3-(4-(2-iodoethyl)-3,5-dimethylphenyl)-5-(1,6,6-trimethyl-4,5,6,7-tetrahydro-1H-indazol-3-yl)-1,2,4-oxadiazole

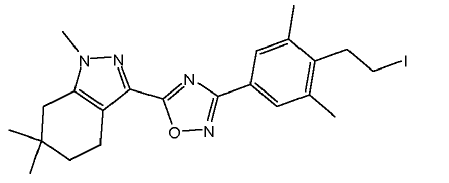

To a suspension of Preparation 28 (229 mg, 0.5 mmol) in acetone, sodium iodide (374.3 mg, 2.5 mmol) was added and mixture stirred overnight to reflux. Solvent was removed, crude redissolved in diethyl ether and washed with saturated NaHCO₃, 10% sodium bisulphite, water and brine. Organic layer was dried over magnesium sulphate, filtered and concentrated. The crude thus obtained was purified by normal phase chromatography using hexane/ethyl acetate 7:3 to yield the title compound (77% yield).
LRMS: m/z 491 (M+1)⁺
Retention time: 8.20 min (Method B)
¹H NMR (300 MHz, DMSO-*d₆*) δ ppm 1.03 (s, 6 H) 1.36 (t, *J*=7.28 Hz, 3 H) 1.58 (t, *J*=6.32 Hz, 2 H) 2.51 (s, 2 H) 2.75 - 2.95 (m, 2 H) 4.17 (q, *J*=7.14 Hz, 2 H) 7.68 - 7.88 (m, 1 H) 7.88 - 8.05 (m, 1 H) 8.21 - 8.46 (m, 2 H).

### Preparation 37

### Diethyl 2,6-dimethyl-4-(5-(1,6,6-trimethyl-4,5,6,7-tetrahydro-1H-indazol-3-yl)-1,2,4-oxadiazol-3-yl)phenethylphosphonate

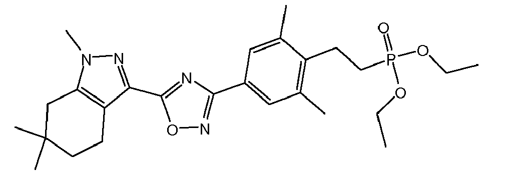

To Preparation 36 (130 mg, 0.27 mmol) triethylphosphite (1.75 mL, 10.2 mmol) was added and mixture heated overnight at 120°C in a sealed tube. Crude was concentrated and purified by normal phase chromatography using hexane/ethyl acetate from 0 to 80% to yield the title compound (100% yield).
LRMS: m/z 501 (M+1)⁺
Retention time: 7.60 min (Method B)

### EXAMPLES

### EXAMPLE 1

### 3-{2-cyclopropyl-4-[5-(1-ethyl-6,6-dimethyl-4,5,6,7-tetrahydro-1H-indazol-3-yl)-1,2,4-oxadiazol-3-yl]phenyl}propanoic acid

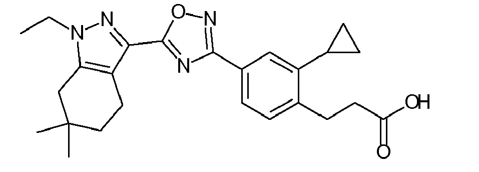

Obtained (32% yield) as sodium salt from Preparation 9 following General Method 3.
LRMS: m/z 435 (M+1)⁺
Retention time: 19.80 min (Method C)
¹H NMR (300 MHz, DMSO-d₆) δ ppm 1.07 (s, 6 H) 1.40 (t, *J*=7.14 Hz, 3 H) 1.61 (t, *J*=5.91 Hz, 2 H) 1.99 - 2.25 (m, 1 H) 2.32 - 2.66 (m, 6 H) 2.84 (t, *J*=5.77 Hz, 2 H) 3.10 (t, *J*=7.83 Hz, 2 H) 4.21 (q, *J*=7.14 Hz, 3 H) 7.42 (d, *J*=7.97 Hz, 1 H) 7.63 (s, 1 H) 7.83 (d, *J*=7.69 Hz, 1 H).

### EXAMPLE 2

### N-1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)glycinamide

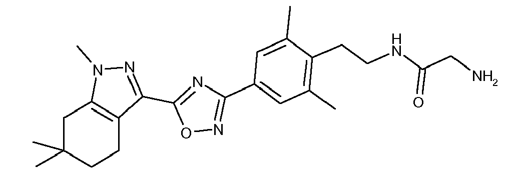

Obtained (67% yield) as hydrochloride salt from Preparation 19 following General Method 4.
LRMS: m/z 437 (M+1)⁺
Retention time: 13.16 min (Method C)
¹H NMR (300 MHz, DMSO-D6) δ ppm 1.1 (m, 6 H) 1.6 (m, 2 H) 2.5 (s, 6 H) 2.5 (s, 2 H) 2.6 (m, 2 H) 2.9 (m, 2 H) 3.3 (m, 2 H) 3.6 (s, 3 H) 3.9 (s, 2 H) 7.8 (s, 2 H) 8.1 (s, 2 H) 8.7 (s, 1 H).

### EXAMPLE 3

### N-1-(2-{2,6-dimethyl-4-[5-(1,6,6-trimethyl-4,5,6,7-tetrahydro-1 H-indazol-3-yl)-1,2,4-oxadiazol-3-yl]phenyl}ethyl)-2-methylalaninamide

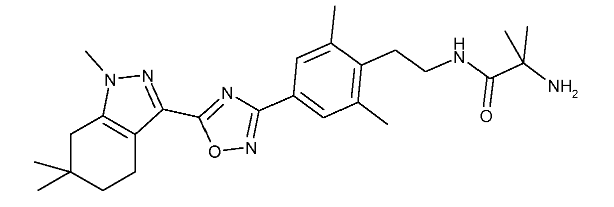

Obtained (93% yield) as hydrochloride salt from Preparation 20 following General Method 4.
LRMS: m/z 465 (M+1)⁺
Retention time: 13.12 min (Method C)
¹H NMR (300 MHz, DMSO-D6) δ ppm 1.0 (s, 6 H) 1.5 (s, 6 H) 1.6 (t, 2 H) 2.5 (m, 6 H) 2.8 (m, 4 H) 3.2 (m, 2 H) 3.6 (s, 2 H) 3.8 (s, 3 H) 7.7 (s, 2 H) 8.2 (s, 2 H) 8.6 (s, 1H).

### EXAMPLES 4 to 12

**N-2-(2-{2,6-dimethyl-4-[5-(1,6,6-trimethyl-4,5,6,7-tetrahydro-1H-indazol-3-yl)-1,2,4-oxadiazol-3-yl]phenyl}ethyl)glycinamide**
**3-(3-{4-[2-(4,4-difluoropiperidin-1-yl)ethyl]-3,5-dimethylphenyl}-1,2,4-oxadiazol-5-yl)-1,6,6-trimethyl-4,5,6,7-tetrahydro-1H-indazole**
**(2-{2,6-dimethyl-4-[5-(1,6,6-trimethyl-4,5,6,7-tetrahydro-1H-indazol-3-yl)-1,2,4-oxadiazol-3-yl]phenyl}ethyl)[2-(1H-1,2,4-triazol-1-yl)ethyl]amine**
**4-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperazin-2-one**
**1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-4-methylpiperidine-4-carboxylic acid**
**N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-serine**
**3-[(2-{2,6-dimethyl-4-[5-(1,6,6-trimethyl-4,5,6,7-tetrahydro-1H-indazol-3-yl)-1,2,4-oxadiazol-3-yl]phenyl}ethyl)amino]-2,2-difluoropropanoic acid**
**1-[(2-{2,6-dimethyl-4-[5-(1,6,6-trimethyl-4,5,6,7-tetrahydro-1H-indazol-3-yl)-1,2,4-oxadiazol-3-yl]phenyl}ethyl)amino]cyclopentanecarboxylic acid**
**{4-[(2-{2,6-dimethyl-4-[5-(1,6,6-trimethyl-4,5,6,7-tetrahydro-1H-indazol-3-yl)-1,2,4-oxadiazol-3-yl]phenyl}ethyl)amino]piperidin-1-yl}acetic acid**

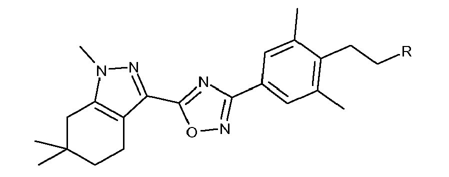

Examples 4 to 12 were obtained as hydrochloride salts from Preparation 17 and the corresponding amino acids following General Method 6.

| **EXAMPLE** | **R** | **tᵣ (Method C) (min)** | **LRMS (M+1)** | **1H NMR δ ppm** |
|---|---|---|---|---|
| 4 | 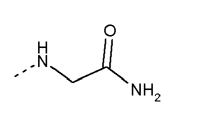 | 12.43 | 437 | ¹H NMR (300 MHz, DMSO-D6) δ ppm 1.0 (s, 6 H) 1.6 (t, *J*=6.5 Hz, 2 H) 2.4 (s, 6 H) 2.5 (m, 6 H) 2.8 (t, *J*=5.8 Hz, 2 H) 3.0 (m, 2 H) 3.8 (s, 3 H) 7.6 (s, 1 H) 7.7 (s, 2 H) 7.9 (s, 1 H) |
| 5 | 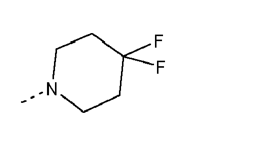 | 14.49 | 483 | ¹H NMR (300 MHz, DMSO-D6) δ ppm 1.0 (s, 6 H) 1.6 (t, *J*=6.2 Hz, 2 H) 2.4 (m, 4 H) 2.5 (s, 6 H) 2.5 (s, 6 H) 2.8 (t, *J*=6.3 Hz, 2 H) 3.2 (m, 4 H)3.8(s,3H)7.8(s,2H) |
| 6 | 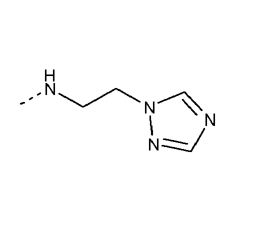 | 12.26 | 475 | ¹H NMR (300 MHz, DMSO-D6) δ ppm 1.0 (s, 6 H) 1.6 (t, *J*=6.0 Hz, 2 H) 2.4 (s, 6 H) 2.5 (t, 2 H) 2.8 (t, *J*=5.9 Hz, 2 H) 3.0 (m, 4 H) 3.5 (m, 2 H) 3.8 (s, 3 H) 4.6 (m, *J*=5.9, 5.9 Hz, 2 H) 7.7 (s, 2 H) 8.1 (s, 1 H) 8.7 (s, 1 H) 9.3 (s, 1 H). |
| 7 | 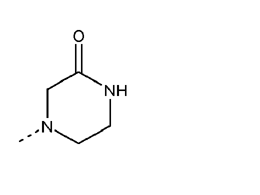 | 15.03 | 463 | ¹H NMR (300 MHz, DMSO-D6) δ ppm 1.0 (s, 6 H) 1.6 (t, *J*=5.9 Hz, 2 H) 2.5 (s, 6 H) 2.5(s,2H)2.5(m,2H)2.8 (t, *J*=5.9 Hz, 2 H) 3.2 (m, 4 H) 3.4 (m, 4 H) 3.8 (s, 3 H) 7.8 (s, 2 H) 8.5 (s, 1 H). |
| 8 | 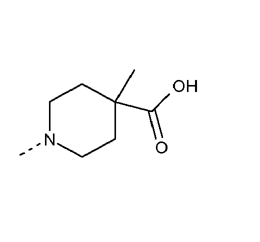 | 13.31 | 506 | ¹H NMR (300 MHz, DMSO-d₆) δ ppm 1.03 (s, 6 H) 1.55 (t, *J*=5.91 Hz, 2 H) 1.66 - 1.91 (m, 2 H) 2.02 - 2.32 (m, 2 H) 2.35 - 2.51 (m, 9 H) 2.80 (t, *J*=5.91 Hz, 2 H) 2.85 - 3.03 (m, 2 H) 3.11 (br. s., 4 H) 3.64 (d, *J*=10.71 Hz, 2 H) 3.83 (s, 3 H) 7.75 (s, 2 H) |
| 9 | 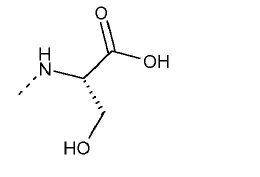 | 15.80 | 468 | ¹H NMR (400 MHz, DMSO-*d₆*) δ ppm 1.02 (s, 6 H) 1.55 (t, J=6.06 Hz, 2 H) 2.26 - 2.53 (m, 8 H) 2.66 - 3.14 (m, 6 H) 3.46 - 3.75 (m, 3 H) 3.83 (s, 3 H) 7.72 (s, 2 H) |
| 10 | 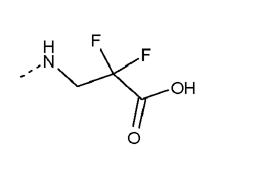 | 16.07 | 488 | ¹H NMR (400 MHz, DMSO-d₆) δ ppm 1.02 (s, 6 H) 1.55 (t, *J*=6.25 Hz, 2 H) 2.42 (s, 6 H) 2.47 (s, 2 H) 2.80 (t, *J*=6.45 Hz, 2 H) 3.07 (m, 4 H) 3.57 (m, 2 H) 3.83 (s, 3 H) 7.74 (s, 2 H) |
| 11 | 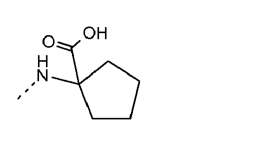 | 15.98 | 492 | ¹H NMR (400 MHz, DMSO-d₆) δ ppm 1.02 (s, 6 H) 1.55 (t, *J*=6.06 Hz, 2 H) 1.65 - 1.90 (m, 2 H) 1.94 - 2.08 (m, 2 H) 2.17 (d, *J*=7.03 Hz, 2 H) 2.43 (s, 6 H) 2.80 (t, *J*=5.67 Hz, 2 H) 2.86 - 3.17 (m, 4 H) 3.83 (s, 3 H) 7.76 (s, 2 H) |
| 12 | 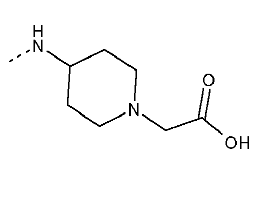 | 11.65 | 521 | ¹H NMR (400 MHz, DMSO-d₆) δ ppm 1.02 (s, 6 H) 1.55 (t, *J*=6.25 Hz, 2 H) 2.04 - 2.30 (m, 4 H) 2.43 - 2.49 (m, 4 H) 2.54 (s, 6 H) 2.80 (t, *J*=6.25 Hz, 2 H) 2.87 - 3.05 (m, 4 H) 3.07 - 3.26 (m, 3 H) 3.83 (s, 3 H) 4.10 (s, 2 H) 7.74 (s, 2 H) 9.93 (br. s., 3 H) |

### EXAMPLE 13

### 2-(2-aminoethoxy)-5-[5-(1,6,6-trimethyl-4,5,6,7-tetrahydro-1H-indazol-3-yl)-1,2,4-oxadiazol-3-yl]benzoic acid

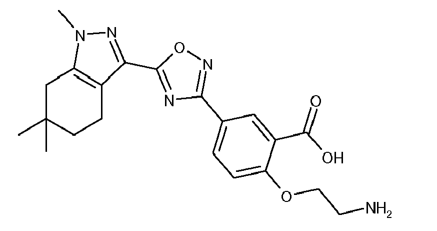

Obtained (100% yield) as hydrochloride salt from Preparation 24 following General Method 4.
LRMS: m/z 412 (M+1)⁺
Retention time: 12.03 min (Method C)
¹H NMR (250 MHz, DMSO-D6) δ ppm 1.01 (s, 6 H) 1.55 (m, 2 H) 2.79 (m, 2 H) 3.27 (m, 2 H) 3.82 (s, 3 H) 4.37 (m, 2 H) 7.42 (d, *J*=8.8 Hz, 1 H) 8.05 (sa, 2 H) 8.22 (d, *J*=7.7 Hz, 1 H) 8.40 (s, 1 H).

### EXAMPLE 14

### 2-(2-aminoethoxy)-5-[5-(1,6,6-trimethyl-4,5,6,7-tetrahydro-1H-indazol-3-yl)-1,2,4-oxadiazol-3-yl]benzamide

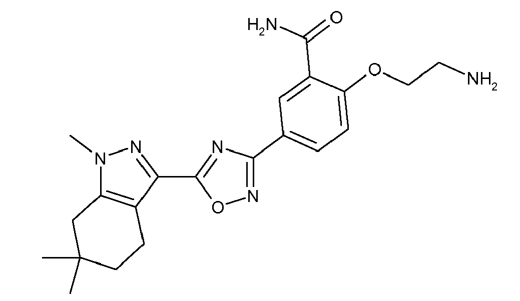

Obtained (44% yield) as hydrochloride salt from Preparation 24 following General Method 5 followed by General Method 4.
LRMS: m/z 411 (M+1)⁺
Retention time: 10.29 min (Method C)
¹H NMR (250 MHz, DMSO-D6) δ ppm 1.01 (s, 6 H) 1.55 (t, *J*=6.3 Hz, 2 H) 2.47 (sa, 2 H) 2.80 (t, *J*=6.3 Hz, 1 H) 3.33 (m, 2 H) 3.83 (s, 3 H) 4.43 (t, *J*=4.9 Hz, 2 H) 7.39 (d, *J*=9.1 Hz, 1 H) 7.77 (sa, 1 H) 7.81 (sa, 1 H) 8.16 (dd, *J*=7.7, 2.1 Hz, 1 H) 8.28 (sa, 1 H) 8.44 (d, *J*=2.1 Hz, 1 H).

### EXAMPLE 15

### 1-{4-[5-(1,6,6-trimethy)-4,5,6,7-tetrahydro-1H-indazol-3-yl)-1,2,4-oxadiazol-3-yl]phenyl}piperidin-4-ol

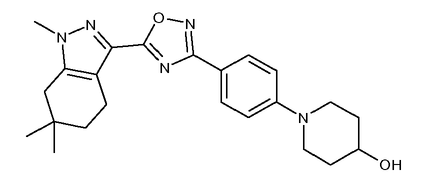

Obtained (5% yield) from Preparation 11 and Preparation 26 following General Method 2.
LRMS: m/z 408 (M+1)⁺
Retention time: 18.12 min (Method C)
¹H NMR (300 MHz, CHLOROFORM-d) δ ppm 1.05 (s, 6 H) 1.42 - 1.93 (m, 6 H) 2.40 (s, 2 H) 2.90 (t, *J*=6.04 Hz, 2 H) 2.97 - 3.19 (m, 2 H) 3.46 - 3.78 (m, 2 H) 3.78 - 4.01 (m, 4 H) 6.99 (d, *J*=9.06 Hz, 2 H) 7.94 - 8.19 (d, *J*=9.06 Hz, 2 H).

### EXAMPLE 16

### 3-(3-{3,5-di methyl-4-[2-(1H-1,2,4-triazol-1-yl)ethyl]phenyl}-1,2,4-oxadiazol-5-yl)-1,6,6-trimethyl-4,5,6,7-tetrahydro-1H-indazole

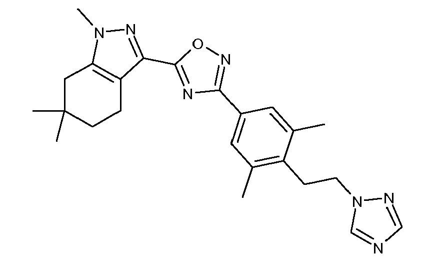

To a solution of 1H-1,2,4-triazole (27.11 mg, 0.39 mmol) in DMF (1 mL), NaH 60% (21 mg, 0.52 mmol) was added and the mixture was stirred at r.t. for 30 min. Then Preparation 28 (120 mg, 0.26 mmol) dissolved in DMF (2.5 mL) was added and reaction heated at 60°C for 3 h. Crude reaction was poured onto ice-water and product extracted with ethyl acetate, organic fractions were combined and washed with brine, dried over magnesium sulphate, filtered and concentrated. The solid thus obtained was purified by normal phase chromatography using hexane/ethyl acetate from 15 to 100% to yield the title compound (59% yield).
LRMS: m/z 432 (M+1)⁺
Retention time: 18.97 min (Method C)
¹H NMR (300 MHz, DMSO-*d₆*) δ ppm 1.02 (s, 6 H) 1.55 (t, *J*=6.18 Hz, 2 H) 2.33 (s, 6 H) 2.47 (s, 2 H) 2.80 (t, *J*=6.04 Hz, 2 H) 3.18 (t, *J*=7.28 Hz, 2 H) 3.83 (s, 3 H) 4.37 (t, *J*=7.28 Hz, 2 H) 7.72 (s, 2 H) 8.01 (s, 1 H) 8.40 (s, 1 H).

### EXAMPLE 17

### 4-{2,6-dimethyl-4-[5-(1,6,6-trimethyl-4,5,6,7-tetrahydro-1H-indazol-3-yl)-1,2,4-oxadiazol-3-yl]phenyl}-2-methylbutan-2-ol

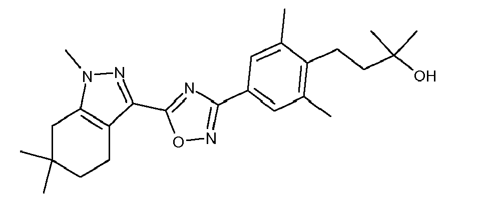

To a solution of Preparation 32 (55 mg, 0.13 mmol) in THF (1.3 mL) under argon atmosphere MeMgBr 3 M in diethyl ether (0.21 mL, 0.63 mmol) was added and the mixture was stirred at 50°C for 2 h. Then saturated ammonium chloride solution was added, organic layer was washed with brine, dried over sodium sulphate, filtered and concentrated. The crude thus obtained was purified by normal phase chromatography using hexane/ethyl acetate 6:4 to yield the title compound as a white solid (58% yield).
LRMS: m/z 423 (M+1)⁺
Retention time: 20.53 min (Method C)
¹H NMR (300 MHz, CHLOROFORM-*d*) δ ppm 1.07 (s, 6 H) 1.35 (s, 6 H) 1.58 (m, 4 H) 2.40 (m, 6 H) 2.63 - 2.82 (m, 2 H) 2.90 (t, *J*=4.81 Hz, 2 H) 3.86 (s, 3 H) 7.87 (s, 2 H).

### EXAMPLE 18

### 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-1H-pyrazol-3-amine

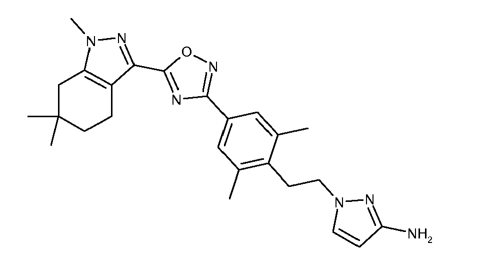

Obtained (10% yield) from Preparation 28 and 1H-pyrazol-3-amine following the procedure described in Example 16.
LRMS: m/z 446 (M+1)⁺
Retention time: 18.40 min (Method C)
¹H NMR (300 MHz, DMSO-*d₆*) δ ppm 1.02 (s, 6 H) 1.55 (t, *J*=6.32 Hz, 2 H) 2.34 (s, 6 H) 2.47 (s, 2 H) 2.80 (t, *J*=5.91 Hz, 2 H) 3.09 (t, 2 H) 3.83 (s, 3 H) 3.97 (t, *J*=7.55 Hz, 2 H) 4.61 (s, 2 H) 5.35 (d, *J*=2.20 Hz, 1 H) 7.19 (d, *J*=2.20 Hz, 1 H) 7.71 (s, 2 H).

### EXAMPLE 19

### 3-(3-{3,5-dimethyl-4-[2-(1H-1,2,4-triazol-3-yl)ethyl]phenyl}-1,2,4-oxadiazol-5-yl)-1,6,6-trimethyl-4,5,6,7-tetrahydro-1H-indazole

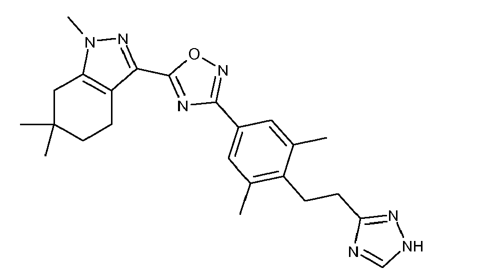

To a solution of formohydrazide (35.3 mg, 0.59 mmol) in ethanol (3 mL) and molecular sieves, triethylamine (1 mL, 18 mmol) was added and incubated for 15 min. Then this mixture was added to Preparation 34 (185 mg, 0.39 mmol) and reaction stirred overnight at 80°C. Solvent was removed and crude was redissolved in DCM and washed with water, dried over magnesium sulphate, filtered and dried. Crude was purified according to General Purification Method followed by normal phase purification using dichloromethane/methanol from 0 to 10% to yield the title compound (23% yield).
LRMS: m/z 432 (M+1)⁺
Retention time: 18.27 min (Method C)
¹H NMR (300 MHz, CHLOROFORM-d) δ ppm 1.09 (s, 6 H) 1.61 (t, 2 H) 2.20 - 2.49 (m, 8 H) 2.92 (t, *J*=6.32 Hz, 2 H) 2.96 - 3.07 (m, 2 H) 3.17 (t, *J*=9.75 Hz, 1 H) 3.88 (s, 3 H) 7.90 (s, 2 H) 8.07 (s, 1 H).

### EXAMPLE 20

### 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-4-fluoropiperidine-4-carboxylic acid

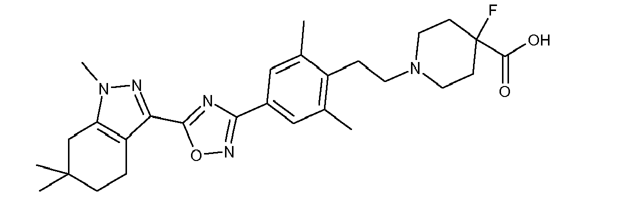

Obtained (34% yield) as hydrochloride salt from Preparation 35 following General Method 3.
LRMS: m/z 510 (M+1)⁺
Retention time: 15.62 min (Method C)
1 H NMR (300 MHz, DMSO-D6) δ ppm 1.0 (s, 6 H) 1.2 (m, *J*=7.1 Hz, 2 H) 1.6 (t, *J*=5.4 Hz, 2 H) 2.2 (m, 2 H) 2.5 (m, 6 H) 2.5 (m, 4 H) 2.8 (t, 2 H) 3.2 (m, 4 H) 3.7 (m, *J*=15.1 Hz, 2 H) 3.8 (s, 3 H) 7.7 (s, 2 H) 11.1 (s, 1 H).

### EXAMPLE 21

### (2-{2,6-dimethyl-4-[5-(1,6,6-trimethyl-4,5,6,7-tetrahydro-1H-indazol-3-yl)-1,2,4-oxadiazol-3-yl]phenyl}ethyl)phosphonic acid

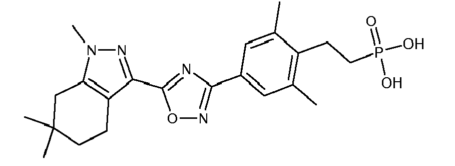

To a solution of Preparation 37 in dichloromethane at 0°C bromotrimethylsilane was added and the mixture was stirred overnight at r.t and then 2 h at 40°C. Solvent was removed, crude was resuspended in 1 N NaOH, washed with diethyl ether and aquous layer acidified with concentrated HCl and product extracted with dichloromethane. Crude was purified according to the General Purification Method to yield the title compound (13% yield).
LRMS: m/z 445 (M+1)⁺
Retention time: 18.18 min (Method C)
¹H NMR (400 MHz, DMSO-*d₆*) δ ppm 1.54 (t, *J*=6.25 Hz, 2 H) 1.57 - 1.70 (m, 2 H) 2.36 (s, 3 H) 2.46 (s, 2 H) 2.69 - 2.93 (m, 4 H) 3.82 (s, 3 H) 7.69 (s, 2 H).

### EXAMPLE 22

### 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-4-carboxylic acid

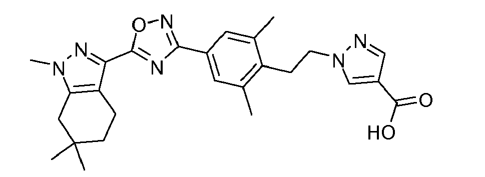

To a solution of Preparation 28 (0.15 g, 0.33 mmol) and 1 H-pyrazole-4-carboxylic acid (55 mg, 0.39 mmol) in DMF (3 mL) NaH (40 mg, 1 mmol) was added and mixture stirred overnight at r.t and for 6h at 60°C. Reaction was stopped by adding 2N HCl and product extracted with dichloromethane. The crude thus obtained was purified according to the General Purification Method to give the title compound (10% yield).
LRMS: m/z 475 (M+1)⁺
Retention time: 19.55 min (Method C)
¹H NMR (300 MHz, DMSO-*d₆*) δ ppm 1.03 (s, 6 H) 1.55 (t, *J*=6.32 Hz, 2 H) 2.34 (s, 6 H) 2.48 (s, 2 H) 2.81 (t, *J*=5.63 Hz, 2 H) 3.19 (m, 2 H) 3.82 (s, 3 H) 4.31 (t, *J*=7.42 Hz, 2 H) 7.73 (s, 2 H) 7.84 (s, 1 H) 8.18 (s, 1 H).

### EXAMPLE 23

### 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-3-carboxylic acid

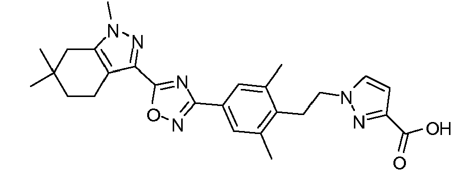

Obtained (11% yield) from Preparation 28 following the procedure described in Example 22.
LRMS: m/z 475 (M+1)⁺
Retention time: 18.83 min (Method C)
¹H NMR (400 MHz, DMSO-*d₆*) δ ppm 1.00 (s, 6 H) 1.53 (t, *J*=6.25 Hz, 2 H) 2.31 (s, 6 H) 2.45 (s, 2 H) 2.78 (t, *J*=6.06 Hz, 2 H) 3.16 (t, *J*=7.42 Hz, 2 H) 3.80 (s, 3 H) 4.33 (t, *J*=7.42 Hz, 2 H) 6.63 (d, *J*=2.34 Hz, 1 H) 7.67 (d, *J*=1.95 Hz, 1 H) 7.70 (s, 2 H).

### EXAMPLE 24

### 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-5-carboxylic acid

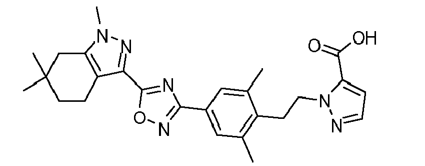

Obtained (13% yield) from Preparation 28 following the procedure described in Example 22.
LRMS: m/z 475 (M+1)⁺
Retention time: 20.05 min (Method C)
¹H NMR (400 MHz, DMSO-*d*₆) δ ppm 1.00 (s, 6 H) 1.53 (t, *J*=5.86 Hz, 2 H) 2.33 (s, 6 H) 2.45 (s, 2 H) 2.78 (t, *J*=5.86 Hz, 2 H) 3.11 (t, *J*=7.42 Hz, 2 H) 3.81 (s, *J*=1.95 Hz, 3 H) 4.65 (t, *J*=7.42 Hz, 2 H) 6.80 (t, *J*=1.76 Hz, 1 H) 7.56 (t, *J*=1.76 Hz, 1 H) 7.69 (s, 2 H).

### PHARMACOLOGICAL ACTIVITY

### 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffers. After drying the filter plate's scintillant liquid were added to each well and 35S-GTPyS binding was measured on a Trilux Scintillation Counter. The results are shown in Table 1.

**Table 1**

| **EXAMPLES** | **EC₅₀ (nM)** |
|---|---|
| 2 | 1.99 |
| 6 | 19.21 |
| 16 | 12.12 |
| 17 | 37.46 |
| 21 | 3.08 |
| 22 | 6.37 |
| 24 | 10.29 |

Compounds of the present invention have high affinities for sphingosine-1-phosphate receptors. Thus the EC₅₀ of the preferred compounds of the invention is lower than 100 nM, preferably less than 50 nM. The most preferred compounds of the invention have an EC₅₀ lower than 20 nM.

The 2-oxadiazole derivatives of the invention may also be combined with other active compounds in the treatment of diseases known to be susceptible to improvement by treatment with a sphingosine-1-phosphate receptor agonist (S1P1 ).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) Interferons comprising Interferon beta 1a such as Avonex from Biogen Idec, CinnoVex from CinnaGen and Rebif from EMD Serono, and Interferon beta 1b such as Betaferon from Schering and Betaseron from Berlex, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide and leflunomide (i) fumaric acid esters, such as *BG-12,* (j) immunomodulators such as Laquinimod, (k) anti-CD20 monoclonal antibodies such as Rituximab, Ocrelizumab Ofatumumab or TRU-015, (I) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as tesocitinib citrate or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162

The combinations of the invention may be used in the treatment of disorders which are susceptible to amelioration by sphingosine-1-phosphate receptors agonists (S1P1 ). Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2 µg and 150 µg of each therapeutically active ingredient. Alternatively, the active ingredient (s) may be presented without excipients.

Packaging of the formulation for inhalation may be carried out by using suitable inhaler devices such as Genuair^{®} (formerly known as Novolizer SD2FL) which is described in the following patent applications: WO 97/000703, WO 03/000325 and WO 03/061742.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-α-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of 3-[(2-{2,6-dimethyl-4-5-(1,6,6-trimethyl-4,5,6,7-tetrahydro-1H-indazol-3-yl)-1,2,4-oxadiazol-3-yl]phenyl}ethyl)amino]-2,2-difluoropropanoic acid (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 3-[(2-{2,6-dimethyl-4-[5-(1,6,6-trimethyl-4,5,6,7-tetrahydro-1H-indazol-3-yl)-1,2,4-oxadiazol-3-yl]phenyl}ethyl)amino]-2,2-difluoropropanoic acid (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I), or a pharmaceutically acceptable salt or N-oxide thereof: 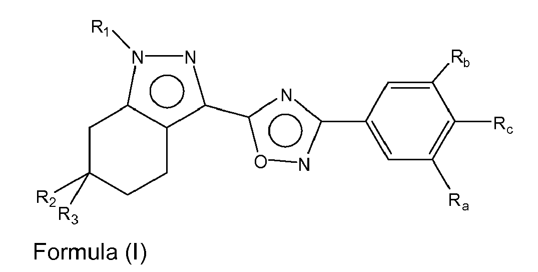 wherein,
R¹ represents a hydrogen atom, a C₁₋₂ alkyl group or a C₃₋₄ cycloalkyl-C₁₋₂ alkyl group,
both R² and R³ represent a C₁₋₂ alkyl group;
R^{a} represents a hydrogen atom or a C₁₋₂ alkyl group;
R^{b} represents a hydrogen atom, a C₁₋₂ alkyl group, a C₃₋₄ cycloalkyl group, a carboxy group, a carbamoyl group or a -CF₃ group;
R^{c} represents a linear or branched C₂₋₅ alkyl group substituted with one substituent selected from a hydroxy group, a triazolyl group and a -P(O)(OH)₂ group; a -(CH₂)₍₁₋₂₎-C(O)OR' group; a -NR'R" group; a -(CH₂)(₁₋₂)-NR'R" group; a -(CH₂)(₁₋₂)-NHC(O)R" group; a -(CH₂)(₂₋₄)-NH-(CH₂)(₁₋₃)-NR'R" group; or a -O-(CH₂)₍₂₋₃₎-NR'R" group, wherein:
R' represents a hydrogen atom or a C₁₋₂ alkyl group,
R" represents a hydrogen atom; a C₅₋₆ cycloalkyl group substituted with a carboxy group; a 5- to 6-membered saturated heterocyclic ring containing one nitrogen atom and substituted with a carboxy(C₁₋₂alkyl) group; or a C₁₋₂ alkyl group optionally substituted by one or more substituents selected from halogen atoms, hydroxy groups, methyl groups, amino groups, carbamoyl groups and carboxy groups; or
R' and R" together with the nitrogen atom to which they are attached form (a) a 4 to 6 membered saturated or unsaturated heterocyclic group, which contains, as heteroatom, one N atom and, optionally, one further N atom and which heterocyclic group is substituted with one or more substituents selected from a halogen atom, a hydroxy group, a methyl group, an oxy group and a carboxy group, or (b) a 5 membered heteroaryl group which contains, as heteroatom, one N atom and, optionally, one or more further N atoms and which heteroaryl group is optionally substituted with one substituent selected from an amino group and a carboxy group.

2. A compound according to claim 1, wherin R¹ represents a methyl or ethyl group, preferably a methyl group.

3. A compound according to any preceding claim, wherein both R² and R³ represent a methyl group.

4. A compound according to any preceding claim wherein R^{a} represents a hydrogen atom or a methyl group, preferably a methyl group

5. A compound according to any preceding claim wherein R^{b} represents a C₁₋₂ alkyl group, a C₃₋₄ cycloalkyl group or a -CF₃ group, preferably a methyl group or a cyclopropyl group, more preferably a methyl group.

6. A compound according to any preceding claim wherein R^{c} represents a linear or branched C₂₋₅ alkyl group substituted with a triazolyl group; a -(CH₂)₂-NR'R" group; a -(CH₂)₂NHC(O)R" group or a -(CH₂)₂-NH-(CH₂)₂-NR'R"group, wherein
R' represents a hydrogen atom,
R" represents a hydrogen atom; a C₅₋₆ cycloalkyl substituted with a carboxy group; or a C₁₋₂ alkyl group optionally substituted by one or more substituents selected from halogen atoms, hydroxy groups, amino groups, carbamoyl groups and carboxy group, or
R' and R" together with the nitrogen atom to which they are attached form (a) a 6 membered saturated or unsaturated heterocyclic group, which contains, as heteroatom, one N atom and, optionally, one further atom selected from N, and which is substituted with one or two substituents selected from halogen atoms, hydroxy groups, methyl groups and carboxy groups, or (b) an unsubstituted triazolyl group.

7. A compound according to claim 6, wherein R^{c} represents a -(CH₂)₂-NR'R" group, R' represents a hydrogen atom and R" represents a C₁₋₂ alkyl group substituted by one or more substituents selected from a fluorine atom, a hydroxy group, a carbamoyl group and a carboxy group

8. A compound according to any preceding claim wherein R¹, R², R³, R^{a} and R^{b} represent methyl groups and R^{c} represents a -(CH₂)₂-NR'R" group, wherein R' represents a hydrogen atom and R" represents a C₁₋₂ alkyl group substituted by one or more substituents selected from fluorine atoms, hydroxy groups, carbamoyl groups and carboxy groups.

9. A compound according to claim 1 wherein: R¹ represents a methyl group or an ethyl group; both R² and R³ represent methyl groups; R^{a} represents a hydrogen atom or a methyl group; R^{b} represents a hydrogen atom, a methyl group, a cyclopropyl group, a carboxy group or a carbamoyl group; R^{c} represents a linear or branched C₂₋₅ alkyl group substituted with one substituent selected from a hydroxy group, a triazolyl group and a -P(O)(OH)₂ group; a -(CH₂)₂-C(O)OR' group; a -NR'R" group; a -(CH₂)₂-NR'R" group, a -(CH₂)₂-NHC(O)R" group; a - (CH₂)₂-NH-(CH₂)₂-NR'R" group or a -O-(CH₂)₂-NR'R" group, wherein:
R' represents a hydrogen atom,
R" represents a hydrogen atom, a cyclopentyl group substituted with a carboxy group, a 6-membered saturated heterocyclic ring containing one nitrogen atom and substituted with a -CH₂COOH group, a C₁₋₂ alkyl group optionally substituted by one or more substituents selected from fluorine atoms, hydroxy groups, methyl groups, amino groups, carbamoyl groups and carboxy groups, or
R' and R" together with the nitrogen atom to which they are attached from (a) a piperidine or piperazine ring which is substituted with one or more substituents selected from a fluorine atom, a methyl group, a hydroxy group, an oxy group and a carboxy group, or (b) a pyrazolyl or triazolyl group which is optionally substituted with one substituent selected from an amino group and a carboxy group.

10. A compound according to claim 1, which is one of
3-{2-Cyclopropyl-4-[5-(1-ethyl-6,6-dimethyl-4,5,6,7-tetrahydro-1H-indazol-3-yl)-1,2,4-oxadiazol-3-yl]phenyl}propanoic acid,
N-1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)glycinamide,
N-1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-2-methylalaninamide,
N-2-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)glycinamide,
3-(3-{4-[2-(4,4-Difluoropiperidin-1-yl)ethyl]-3,5-dimethylphenyl}-1,2,4-oxadiazol-5-yl)-1,6,6-trimethyl-4,5,6,7-tetrahydro-1H-indazole,
(2-{2,6-Dimethyl-4-[5-(1,6,6-trimethyl-4,5,6,7-tetrahydro-1H-indazol-3-yl)-1,2,4-oxadiazol-3-yl]phenyl}ethyl)[2-(1H-1,2,4-triazol-1-yl)ethyl]amine,
4-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)piperazin-2-one,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-4-methylpiperidine-4-carboxylicacid,
N-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-L-serine,
3-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]-2,2-difluoropropanoicacid,
1-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]cyclopentanecarboxylic acid,
{4-[(2-{2,6-Dimethyl-4-[5-(1,6,6-trimethyl-4,5,6,7-tetrahydro-1H-indazol-3-yl)-1,2,4-oxadiazol-3-yl]phenyl}ethyl)amino]piperidin-1-yl}acetic acid,
2-(2-Aminoethoxy)-5-[5-(1,6,6-trimethyl-4,5,6,7-tetrahydro-1H-indazol-3-yl)-1,2,4-oxadiazol-3-yl]benzoic acid,
2-(2-Aminoethoxy)-5-[5-(1,6,6-trimethyl-4,5,6,7-tetrahydro-1H-indazol-3-yl)-1,2,4-oxadiazol-3-yl]benzamide,
1-{4-[5-(1,6,6-Trimethyl-4,5,6,7-tetrahydro-1H-indazol-3-yl)-1,2,4-oxadiazol-3-yl]phenyl}piperidin-4-ol,
3-(3-{3,5-Dimethyl-4-[2-(1H-1,2,4-triazol-1-yl)ethyl]phenyl}-1,2,4-oxadiazol-5-yl)-1,6,6-trimethyl-4,5,6,7-tetrahydro-1H-indazole,
4-{2,6-Dimethyl-4-[5-(1,6,6-trimethyl-4,5,6,7-tetrahydro-1H-indazol-3-yl)-1,2,4-oxadiazol-3-yl]phenyl}-2-methylbutan-2-ol,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazol-3-amine,
3-(3-{3,5-Dimethyl-4-[2-(1H-1,2,4-triazol-3-yl)ethyl]phenyl}-1,2,4-oxadiazol-5-yl)-1,6,6-trimethyl-4,5,6,7-tetrahydro-1H-indazole,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-4-fluoropiperidine-4-carboxylicacid,
(2-{2,6-Dimethyl-4-[5-(1,6,6-trimethyl-4,5,6,7-tetrahydro-1H-indazol-3-yl)-1,2,4-oxadiazol-3-yl]phenyl}ethyl)phosphonic acid,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-4-carboxylic acid,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-3-carboxylic acid,
1-(2-{2,6-Dimethyl-4-[5-(1,6,6-trimethyl-4,5,6,7-tetrahydro-1H-indazol-3-yl)-1,2,4-oxadiazol-3-yl]phenyl}ethyl)-1H-pyrazole-5-carboxylic acid,
and a pharmaceutically acceptable salt or N-oxide thereof.

11. A compound according to any one of claims 1 to 10 for use in the treatment of the human or animal body by therapy.

12. A compound according to any one of claims 1 to 10 for use in the treatment of a pathological condition or disease susceptible to amelioration by sphingosine-1-phosphate receptors (S1P1) agonists.

13. A compound for use according to claim 12, wherein the pathological condition or disease is selected from autoimmune diseases, chronic immune and inflammatory diseases, transplant rejection, malignant neoplastic diseases, angiogenic-related disorders, pain, neurological diseases,viral and infectious diseases.

14. A compound for use according to claim 13 wherein the pathological condition or disease is selected from multiple sclerosis, transplant rejection, systemic lupus erythematosus, asthma, psoriasis, rheumatoid arthritis, psoriatic arthritis and Crohn's disease,

15. A pharmaceutical composition comprising a compound as defined in any one of claims 1 to 10 in association with a pharmaceutically acceptable diluent or carrier.

16. Use of a compound as defined in any one of claims 1 to 10 in the manufacture of a medicament for the treatment of a pathological condition or disease as defined in claims 12 to 14.

17. A method for treating a subject afflicted with a pathological condition or disease as defined in claims 12 to 14, which comprises administering to said subject an effective amount of a compound as defined in any one of claims 1 to 10.

18. A combination product comprising (i) a compound according to any one of claims 1 to 10; and (ii) another compound selected from:
a) Interferons comprising Interferon beta 1a such as Avonex from Biogen Idec, CinnoVex from CinnaGen and Rebif from EMD Serono, and Interferon beta 1b such as Betaferon from Schering and Betaseron from Berlex,
b) Immunomodulators such as glatiramer acetate,
c) Inhibitors of DNA synthesis and repair, such as Mitoxantrone,
d) Anti-alpha 4 integrin antibodies, such as Natalizumab (Tysabri),
e) Alpha 4 integrin antagonists such as R-1295 , TBC-4746, CDP-323, ELND-002, Firategrast and TMC-2003,
f) Dyhydrofolate reductase inhibitors, such as Methotrexate or CH-1504,
g) Glucocorticoids such as prednisone or methylprednisolone,
h) DHODH inhibitors such as teriflunomide and leflunomide,
*i)* Fumaric acid esters, such as BG-12,
*j*) Immunomodulators such as Laquinimod,
k) Anti-CD20 monoclonal antibodies such as Rituximab, Ocrelizumab Ofatumumab or TRU-015,
l) Anti-CD52 such as alemtuzumab,
m) Anti-CD25 such as daclizumab,
n) Anti-CD88, such as eculizumab or pexilizumab,
o) Calcineurin inhibitors such as cyclosporine A or tacrolimus,
p) IMPDH inhibitors, such as mycophenolate mophetyl,
q) Cannabinoid receptor agonists such as Sativex,
r) Chemokine CCR1 antagonists such as MLN-3897 or PS-031291,
s) Chemokine CCR2 antagonists such as INCB-8696,
t) Interferon alpha such as Sumiferon MP,
u) NF-kappaB activation inhibitors such as FAE and MLN-0415,
v) JAK inhibitors such as tasocitinib citrate or INCB018424,
w) Syk inhibitors, such as R-112,
x) PKC inhibitors, such as NVP-AEB071,
y) Phosphosdiesterase IV inhibitors such as GRC-4039,
z) P38 Inhibitors such as ARRY-797, and
aa) MEK inhibitors, such as ARRY-142886 or ARRY-438162,
